# EUROPEAN PATENT APPLICATION

(11) **EP 4 357 363 A1**
(43) Date of publication of application: **24.04.2024**
(21) Application number: 22825362.1
(22) Date of filing: 16.06.2022
(51) Int. Cl.: C07K 16/28, G01N 33/68, A61P 35/00, A61K 39/00

(54) **ANTI-TM4SF4 HUMANIZED ANTIBODY AND USE THEREOF**

(30) Priority: 18.06.2021 KR 20210079629
(71) Applicant: Korea Atomic Energy Research Institute, Daejeon 34057 (KR); INDUSTRY ACADEMY COOPERATION FOUNDATION OF SEJONG UNIVERSITY, Seoul 05006 (KR)
(72) Inventor: KIM, In Gyu, Daejeon 34140 (KR); RYU, Chun Jeih, Seoul 06597 (KR); KIM, Min Kyu, Seoul 04776 (KR); KIM, Rae Kwon, Sejong 30100 (KR); PARK, Hwangseo, Seoul 04731 (KR); CHOI, Mun Ju, Seoul 02152 (KR); KAHM, Yeon Jee, Hwaseong-si, Gyeonggi-do 18452 (KR); SHIN, Byung Chul, Seoul 04355 (KR); JUNG, U Hee, Namyangju-si, Gyeonggi-do 12095 (KR)
(74) Representative: Cabinet Beau de Loménie
(86) International application number: PCT/KR2022/008570
(87) International publication number: WO 2022/265439

(57) **Abstract**

The present invention relates to an anti-TM4SF4(TransMembrane 4 Superfamily Member 4) humanized antibody and use thereof. The anti-TM4SF4 humanized antibody of present invention has high affinity for binding specifically to TM4SF4 while exhibiting low immunogenicity in humans, and thus can be advantageously used to detect TM4SF4 or to target cancer cells and cancer stem cells that overexpress TM4SF4.

## Description

### [Technical Field]

The present invention relates to a humanized antibody that can specifically bind to TM4SF4(TransMembrane 4 Superfamily Member 4) with high affinity and has low immunogenicity in human, and a composition comprising thereof to prevent or treat cancer.

### [Background Art]

TM4SF4 (TransMembrane 4 Superfamily Member 4) is a type of tetraspanin protein. Other proteins in this class, TM4SF1 and TM4SF5, have been reported to be up-regulated in expression in a number of tumors and involved in epithelial-mesenchymal transition and cell migration, and many cancer cell-related studies have been conducted. Some reports have shown that TM4SF4 is involved in apoptosis, differentiation, and cell invasion ability in cancer cells. Recently, the research team of the present invention reported that TM4SF4 protein promotes cancer stem cell growth, self-renewal ability, and metastasis/infiltration in human lung cancer cells, and demonstrated that TM4SF4 promotes the activation of IGF1Rβ/AKT/NFκB or JAK2 (or FAK)/STAT3, which are important signaling systems in cancer development, and thereby enhances cancer stem cell characteristics through cytokine secretion, thereby making the tumor more malignant. (Choi SI et al., Oncotarget. 2014; 5 (20):9823-9837, Choi SI et al., Oncotarget. 2017; 8 (60):101284-101297).

Antibodies are used as therapeutic agents due to their high binding specificity to target antigens and stability in the human body. In particular, anti-cancer antibodies are being used to greatly improve cancer treatment efficacy by improving them into humanized antibodies, single-chain antibodies, double antibodies, and drug-fusion antibodies based on the development of antibody engineering technology. However, due to the diversity of cancer characteristics and the induction of treatment resistance by expression of new antigens, limitations are being pointed out in the types of antigens that are currently used for targeting cancer cells, and research is continuing to explore new cancer-specific antigens and derive antibodies against them.

In particular, in the case of cancer that is resistant to targeted drugs or radiotherapy used in existing cancer treatments and recurs, it has been reported that the characteristics of cancer stem cells play an important role, and discovering antigens and securing specific antibodies that can be used to target cancer stem cells are emerging as important.

Meanwhile, in order to develop a monoclonal antibody that specifically binds to a specific antigen, the method of injecting the antigen into an animal other than a human and using the antibody produced through the animal's immune system is mainly used. However, since the antibody produced through the above method is not a human protein in that it is derived from an animal other than a human, problems with immunogenicity may occur when administered to the human body. That is, when antibodies derived from species other than humans are administered to the human body, they may induce the generation of HAMA (Human Anti-Mouse Antibody), and the therapeutic efficacy of the heterologous antibodies may be reduced.

Accordingly, a method is proposed to solve the problem described above by obtaining the sequence that plays an important role in binding to the antigen among the amino acid sequences constituting antibodies derived from species other than humans, and replacing the remaining part with the sequence of a human antibody. However, because chimeric antibodies or humanized antibodies produced through the method are a fusion of two different protein regions, there is a possibility that they may not function as antibodies or that their binding affinity for antigens may be reduced. Therefore, it is still difficult to develop a humanized antibody that shows high specificity and affinity for the antigen without inducing the generation of HAMA in the human body.

### [Disclosure]

### [Technical Problem]

An object of the present invention is to provide a novel humanized antibody or an antigen binding fragment thereof that can specifically bind with high affinity to the TM4SF4 (TransMembrane 4 Superfamily Member 4) protein that is over-expressed on the surface of cancer cells, but can also exhibit low immunogenicity when administered to the human body.

Further, an object of the present invention is to provide a polynucleotide, an expression vector and a host cell capable of encoding and expressing the humanized antibody or an antigen binding fragment, and a method for producing the humanized antibody or an antigen binding fragment thereof. Further, the present invention provides a method for producing an antibody or antigen binding fragment thereof, comprising culturing the host cell.

Further, an object of the present invention is to provide a composition and a kit for detecting TM4SF4, and a method for detecting TM4SF4.

Further, an object of the present invention is to provide a pharmaceutical composition for preventing or treating cancer, a composition for inhibiting the growth of cancer stem cells, and a composition for assisting radiation anticancer treatment.

### [Technical Solution]

In order to achieve the above objects, the present invention provides a humanized antibody or an antigen binding fragment thereof, which comprises: a heavy chain variable region comprising FR-H1 having an amino acid sequence of SEQ ID NO: 1, FR-H2 having an amino acid sequence of SEQ ID NO: 2 or an amino acid sequence of SEQ ID NO: 3, FR-H3 having an amino acid sequence of SEQ ID NO: 4 and FR-H4 having an amino acid sequence of SEQ ID NO: 5; and a light chain variable region comprising FR-L1 having an amino acid sequence of SEQ ID NO: 6, FR-L2 having an amino acid sequence of SEQ ID NO: 7, FR-L3 having an amino acid sequence of SEQ ID NO: 8 and FR-L4 having an amino acid sequence of SEQ ID NO: 9, and specifically binds to TM4SF4 (TransMembrane 4 Superfamily Member 4).

Another aspect of the present invention provides a polynucleotide comprising a base sequence encoding the humanized antibody or an antigen binding fragment thereof, an expression vector containing the polynucleotide, and a host cell containing the expression vector.

Another aspect of the present invention provides a composition for detecting TM4SF4 comprising the humanized antibody or an antigen binding fragment thereof and a kit for detecting TM4SF4 comprising the composition for detecting TM4SF4.

Another aspect of the present invention provides a method for detecting TM4SF4, which includes the step of contacting the humanized antibody or an antigen binding fragment thereof with a sample to be detected that is expected to contain TM4SF4.

Another aspect of the present invention provides a pharmaceutical composition for prevention or treatment of cancer which comprises the humanized antibody or an antigen binding fragment thereof, a composition for inhibiting the growth of cancer stem cells, and a composition for assisting radiation anticancer treatment.

### [Advantageous Effects]

Since the humanized antibody of the present invention can specifically bind to TM4SF4 without binding to substances such as BSA, it can be usefully used to detect TM4SF4 or target cancer cells or cancer stem cells that overexpress TM4SF4. In particular, the humanized antibody of the present invention is similar to a mouse-derived antibody or has a significantly higher binding affinity compared to a chimeric antibody, and thus shows excellent effectiveness.

In addition, in the humanized antibody of the present invention, except for most of the CDR sequences, the remaining sequences use amino acid sequences derived from human antibodies or is a modified part of them, therefore, even when administered to the human body, there is a low possibility of inducing HAMA (Human Anti-Mouse Antibody), so the immunogenicity is also low. Accordingly, the humanized antibody of the present invention has the advantage of being able to solve problems with immune responses that may occur when using mouse-derived antibodies.

However, the effects of the present invention are not limited to the effects mentioned above, and other effects not mentioned will be clearly understood by those skilled in the art from the following description.

### [Description of Drawings]

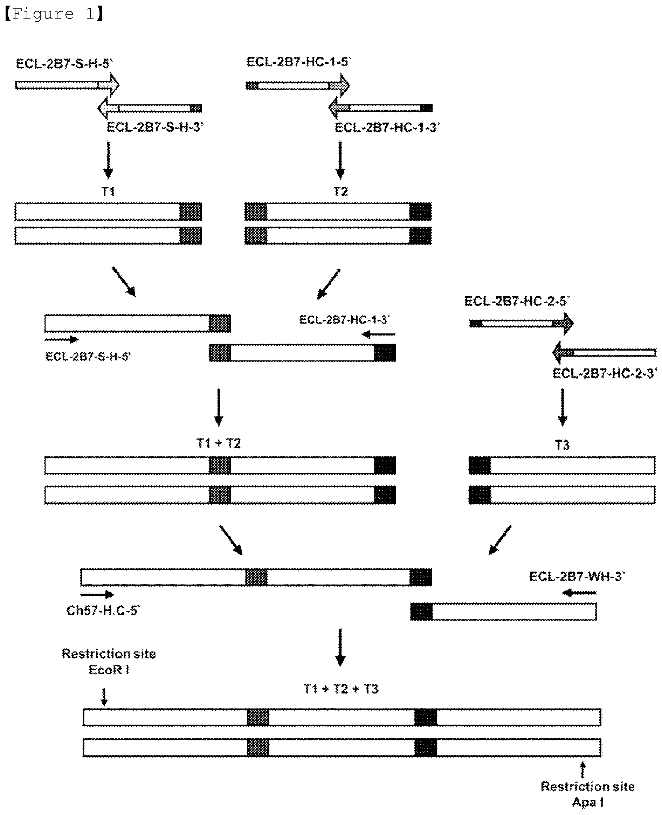

Fig. 1 is a schematic diagram explaining the recombinant PCR process for producing a gene sequence encoding a heavy chain variable region called Hz2B7-1.0 among the humanized antibodies of the present invention.

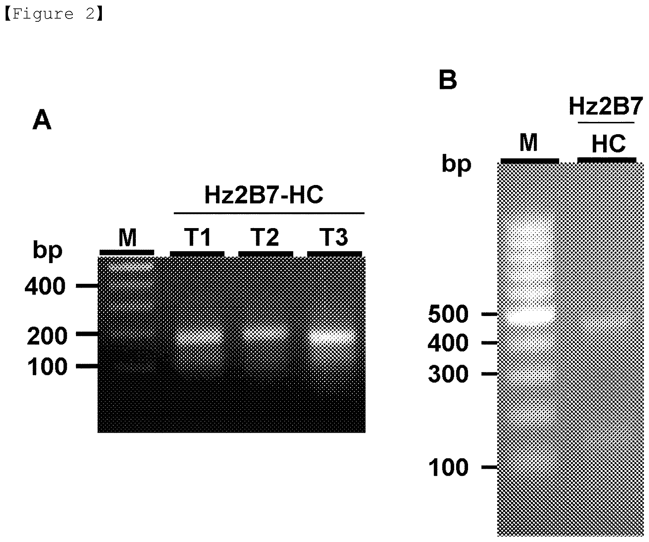

Fig. 2 shows the results of manufacturing the Hz2B7-1.0 heavy chain variable region gene: A shows the results of agarose gel electrophoresis for T1, T2, and T3 fragments, and B shows the electrophoresis results of DNA containing the Hz2B7-1.0 heavy chain variable region gene, which was completed by connecting T, T2, and T3.

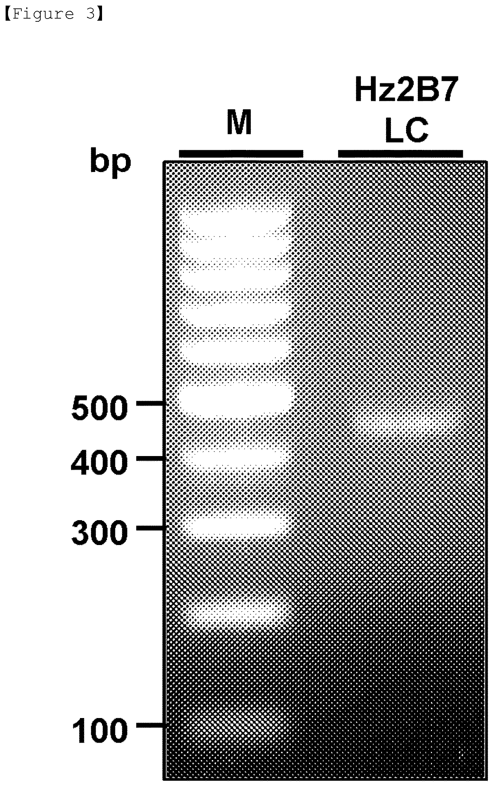

Fig. 3 shows the results of agarose gel electrophoresis of DNA containing a gene encoding a light chain variable region called Hz2B7-0.1 among the humanized antibodies of the present invention.

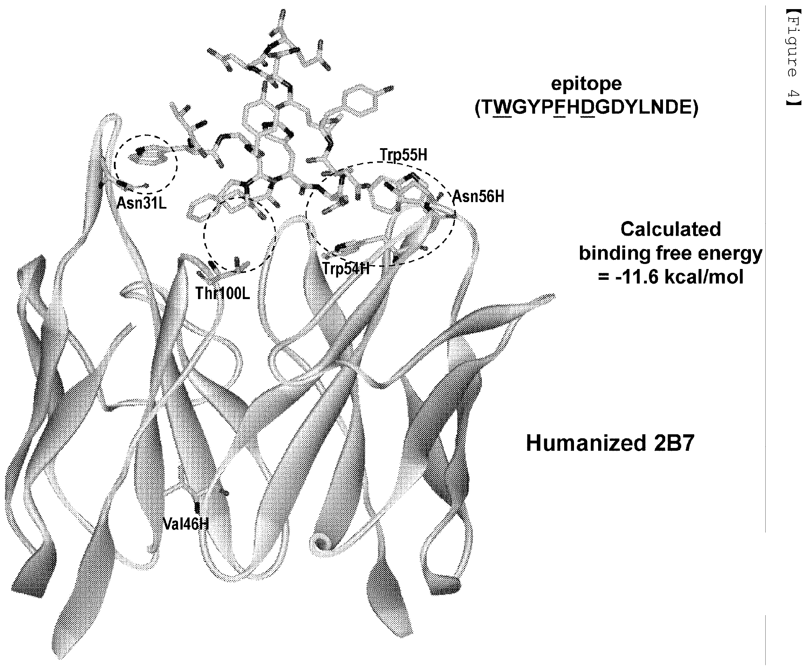

Fig. 4 is the result of analyzing interaction through docking simulation based on the amino acid sequence of Hz2B7-1.1 antibody, a humanized antibody of the present invention, and the epitope sequence of TM4SF4, an antigen. The major amino acid residues of the antibody interacting with the epitope and binding free energy values are indicated. Among the indicated antibody atoms, pink indicates carbon, blue indicates nitrogen, and red indicates oxygen. The carbon atoms of the epitope are shown in green.

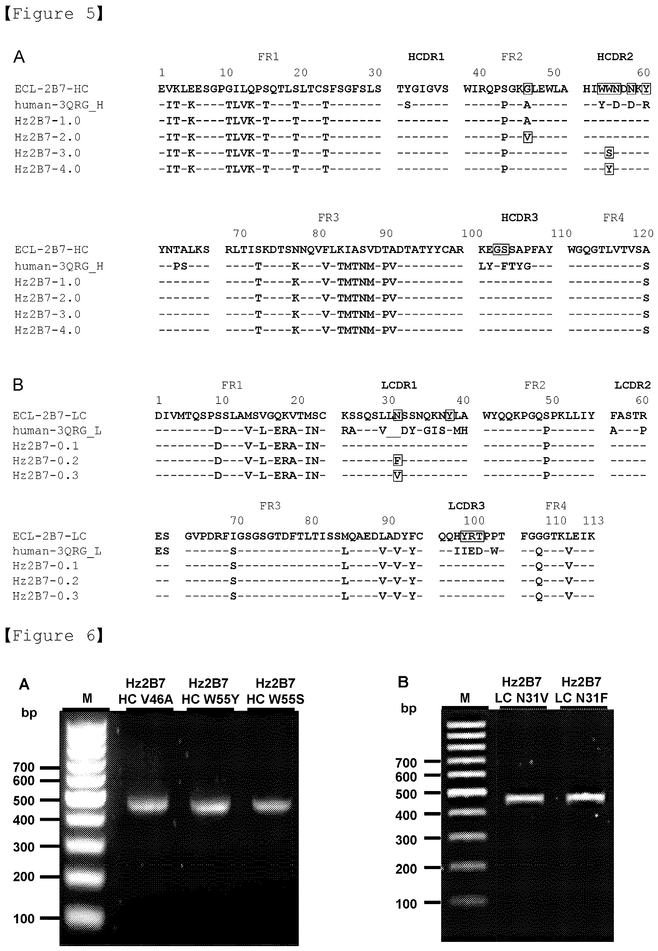

Fig. 5 is a picture comparing the amino acid sequences of a heavy chain (A) and a light chain (B) of the original mouse antibody (2B7) and a similar human antibody (human-3QRG) and the amino acid sequences of 4 heavy chains and 3 light chains of the newly manufactured humanized antibody Hz2B7, in the process of manufacturing the Hz2B7 antibody. The hyphen (-) indicates the same amino acid as the original mouse antibody, and the box indicates the position of the amino acid found to be important for binding to the epitope in this invention.
Fig. 6A shows the results of agarose gel electrophoresis of DNA containing genes of a heavy chain variable region(Hz2B7 HC V46A) called Hz2B7-2.0, a heavy chain variable region(Hz2B7 HC W55Y) called Hz2B7-3.0, and a heavy chain variable region(Hz2B7 HC W55S) called Hz2B7-4.0, among the humanized antibodies of the present invention. Fig. 6B shows the results of agarose gel electrophoresis of DNA containing genes of a light chain variable region(Hz2B7 LC N31V) called Hz2B7-0.2, and a light chain variable region(Hz2B7 LC N31F) called Hz2B7-0.3.

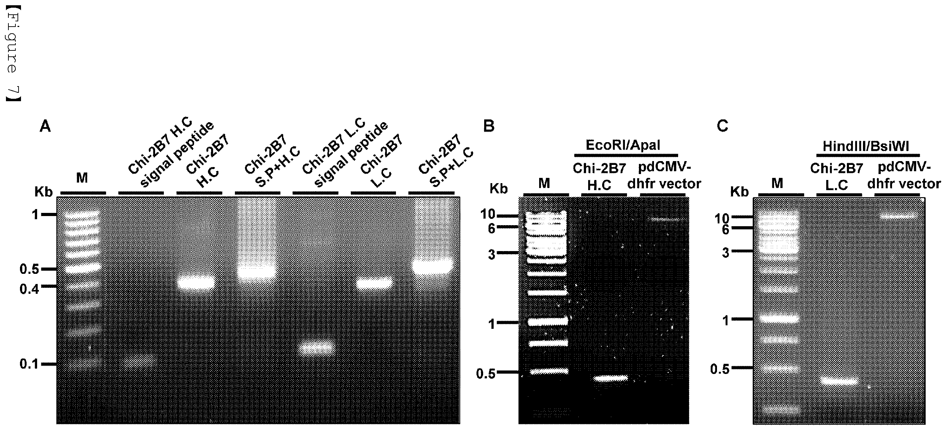

Fig. 7 shows the agarose gel electrophoresis results of the heavy chain and light chain genes of the chimeric antibody used as a comparative example (A), the electrophoresis results of a vector containing the heavy chain gene (B), and the electrophoresis results of a vector containing the light chain gene (C).

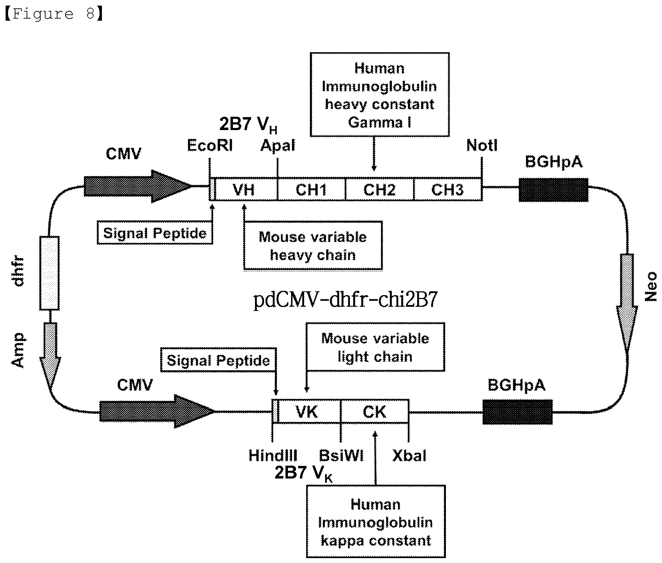

Fig. 8 shows the structure of a vector containing the gene of the chimeric antibody.

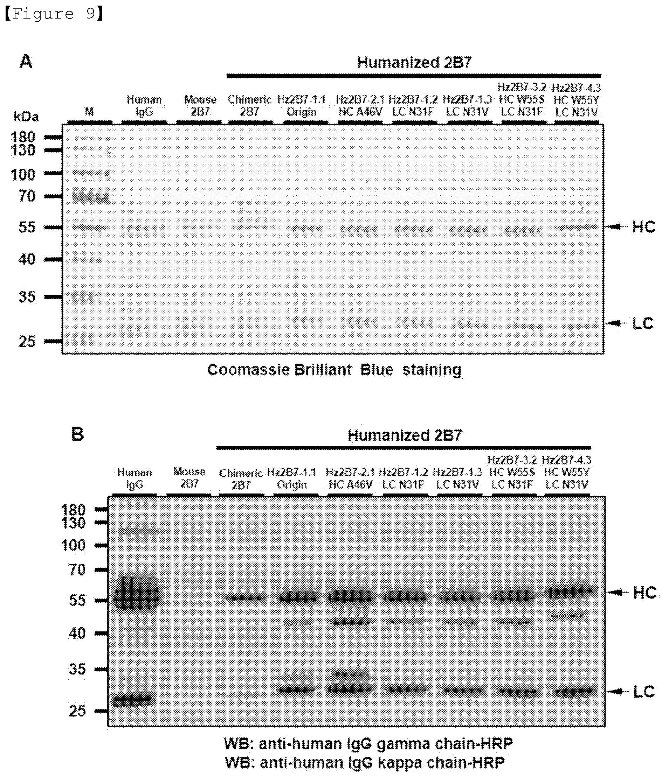

Fig. 9A shows the results of SDS-PAGE and Coomassie blue staining for the humanized antibodies of the present invention, chimeric antibody, mouse-derived 2B7 antibody, and human antibody IgG. Fig. 9B shows the results of Western blotting on the above antibodies, and the secondary antibody used was one that binds to the IgG gamma chain and kappa chain of a human antibody.

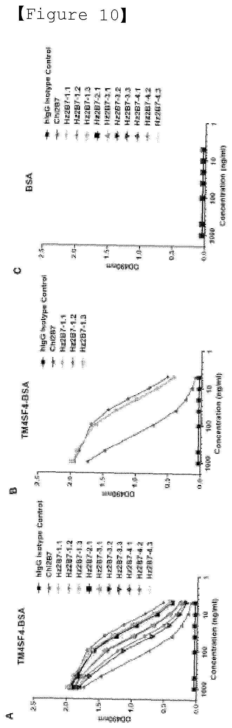

Fig. 10 shows the results of indirect ELISA using 10 types of humanized antibodies of the present invention, chimeric antibody (Chi2B7), and human IgG (Isotype hIgG): A is a graph showing the binding affinity of all antibodies to the TM4SF4-BSA antigen, B is a graph showing the binding affinity of Hz2B7-1.1, Hz2B7-1.2, and Hz2B7-1.3, which show higher binding affinity among the humanized antibodies, and C is a graph showing the binding affinity of all antibodies to BSA.

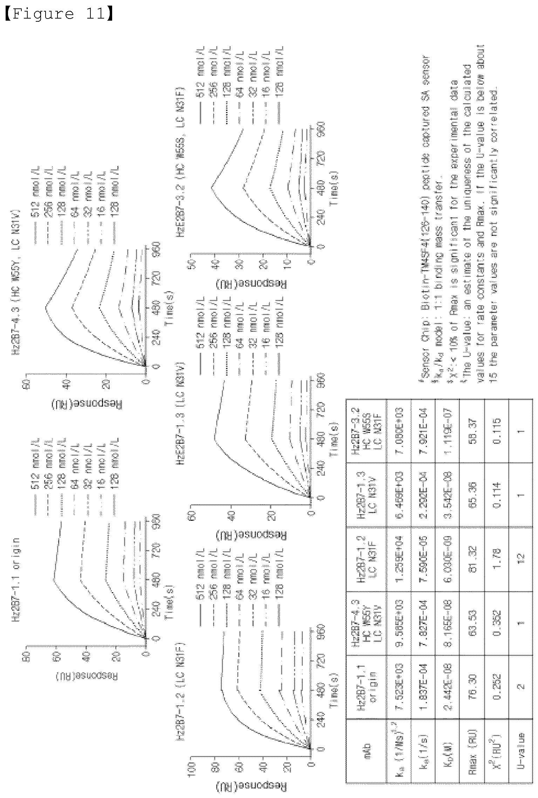

Fig. 11 is a comparative analysis of the antibody affinity for the TM4SF4 protein epitope of five anti-TM4SF4 humanized antibodies using surface plasmon resonance (SPR) analysis. First, biotin-conjugated TM4SF4 peptide was attached to a sensor chip, five types of humanized antibodies were flowed, and the association rate (Ka), dissociation rate (Kd), and equilibrium dissociation constant (KD, Kd/Ka) values of the antibodies were calculated.

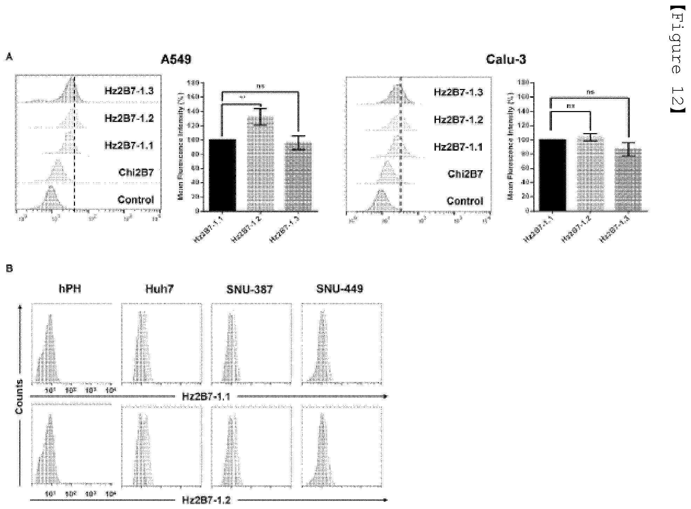

Fig. 12 shows the results of FACS analysis using a flow cytometer on lung cancer, human primary liver cells, and liver cancer cell lines using the humanized antibody of the present invention (Hz2B7-1.1, Hz2B7 (Hz2B7-1.1, Hz2B7-1.2, Hz2B7-1.3), chimeric antibody (Chi2B7), and human IgG of the present invention: A is a graph comparing the binding capacity of antibodies in lung cancer cell lines A549 cells and Calu-3 cell lines, and B is a graph confirming the binding capacity of Hz2B7-1.1 and Hz2B7-1.2 antibodies in human primary liver cells (hPH) and liver cancer cell lines Huh-7, SNU-387, and SNU-449 cell lines.

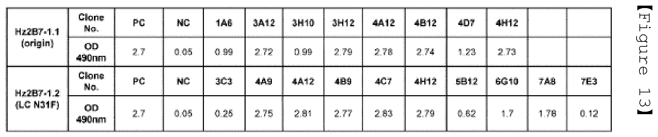

Fig. 13 shows that CHO-DG44 cells were transformed by introducing the gene encoding the humanized antibody of the present invention, then clones with G418 resistance were selected, and the antibody production of each clone was compared by measuring the OD value through ELISA. As a positive control, 1 µg of human IgG was used.

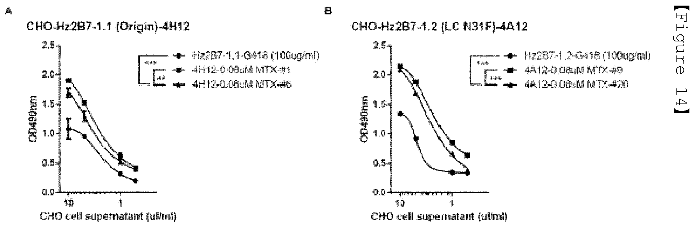

Fig. 14 shows a comparison of the antibody production through sandwich ELISA for the Hz2B7-1.1-4H12 clone and Hz2B7-1.2-4A12 clone whose antibody genes were amplified through MTX: A shows a comparison of the antibody production of the Hz2B7-1.1-4H12 clone without MTX treatment and the clone treated with 0.08 µM MTX, and B shows a comparison of the antibody production of the Hz2B7-1.2-4A12 clone.

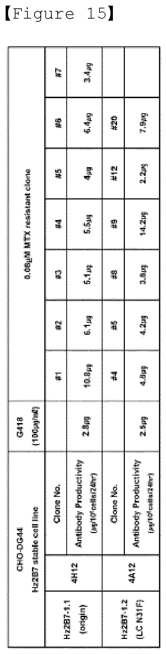

Fig. 15 shows a comparison of the antibody production (µg/10⁶cell/24hr) of the Hz2B7-1.1-4H12 clone and Hz2B7-1.2-4A12 clone, whose antibody genes were amplified by treatment with 0.08 µM MTX, measured using sandwich ELISA.

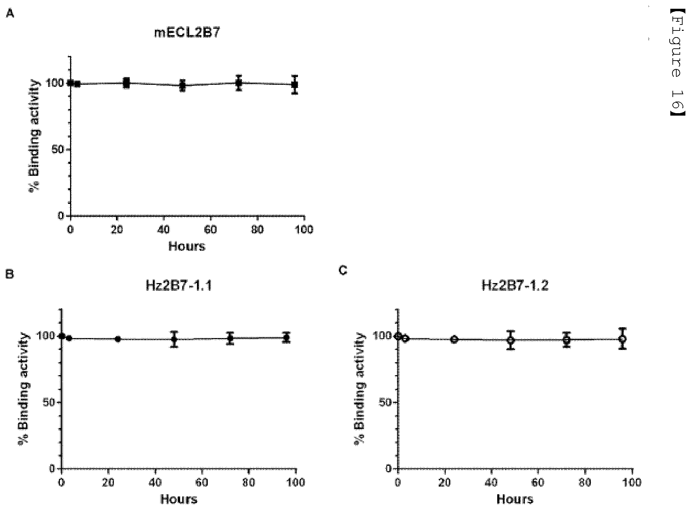

Fig. 16 is a graph measuring and comparing the stability of mouse and humanized antibodies in human serum.

### [Best Mode]

Hereinafter, the present invention will be described in detail.

### 1. Humanized antibody specifically binding to TM4SF4 and an antigen binding fragment thereof

One aspect of the present invention provides a humanized antibody or an antigen binding fragment thereof that specifically binds to TM4SF4 and shows low immunogenicity.

In the present invention, the term "antibody" refers to an immunoglobulin molecule that is immunologically reactive by specifically binding to the epitope of an antigen. The antibody may include a monoclonal antibody, a polyclonal antibody, an antibody with a full-length chain structure (full-length antibody), a functional fragment with at least an antigen binding function (antigen binding fragment), and a recombinant antibody. Specifically, the antibody of the present invention may be a monoclonal antibody or an antigen binding fragment thereof. The monoclonal antibody refers to an antibody molecule with a single molecule composition obtained from a substantially identical antibody population, and this monoclonal antibody exhibits single binding specificity and affinity for a specific epitope. The full-length antibody has a structure of two full-length light chains and two full-length heavy chains, and each light chain may be connected to the heavy chain through a disulfide bond. The antibody includes heavy chain (HC) and light chain (LC) polypeptides, and the heavy chain and light chain may include a variable region and a constant region.

The constant region is a region that mediates binding of the antibody to various types of cells of the immune system (T-cells, etc.), host tissues containing components of the complement system, etc. The constant region performs the same function regardless of the type of antigen if it is the same type of antibody derived from the same species, and the amino acid sequence forming it also has the same or higher degree of similarity for each antibody. The constant region can be divided into a heavy chain constant region (abbreviated as CH) and a light chain constant region (abbreviated as CL). The heavy chain constant region has gamma (γ), mu (µ), alpha (α), delta (δ) and/or epsilon (ε) types, and has subclasses of gamma 1 (γ1), gamma 2 (γ2), gamma 3 (γ3), gamma 4 (γ4), alpha 1 (α1), and/or alpha 2 (α2). The light chain constant region has kappa (κ) and lambda (λ) types. IgG subtypes include IgG1, IgG2, IgG3, and IgG4.

The variable region is an antibody region that has specificity for an antigen and can be divided into a heavy chain variable region (can be abbreviated as VH) and a light chain variable region (can be abbreviated as VL). The variable region may include three complementarity-determining regions (CDRs) and four framework regions (FRs). The CDR may be a ring-shaped region involved in antigen recognition, and specificity for the antigen may be determined depending on the amino acid sequence of the CDR. The CDRs may be referred to as CDR1, CDR2, and CDR3 in their order. The CDR may be referred to as CDR-H1, CDR-H2, or CDR-H3 in the case of heavy chain variable region, and as CDR-L1, CDR-L2, and CDR-L3 in the case of a light chain variable region, depending on whether it is a CDR of a heavy chain or light chain polypeptide. Likewise, FR may be referred to as FR-H1, FR-H2, FR-H3, and FR-H4 for heavy chain variable regions, and as FR-L1, FR-L2, FR-L3, and FR-L4 for light chain variable regions. Additionally, the CDRs and FRs may be arranged in the following order in each variable region. The order is from N-terminus (amino-terminus) to C-terminus (carboxy-terminus): FR-H1, CDR-H1, FR-H2, CDR-H2, FR-H3, CDR-H3, FR-H4 for heavy chain variable region, FR-L1, CDR-L1, FR-L2, CDR-L2, FR-L3, CDR-L3, FR-L4 for light chain variable region.

In the present invention, the term "antigen binding fragment" refers to any fragment of the humanized antibody of the present invention that retains the antigen binding function of the antibody. The antigen binding fragment may be referred to interchangeably with terms such as "fragment" and "antibody fragment," and the antigen binding fragment may be Fab, Fab', F(ab')₂, Fv, etc., but is not limited thereto.

The Fab has a structure that includes the variable regions of the light chain and heavy chain, the constant region of the light chain and the first constant region (CH1 domain) of the heavy chain, and has one antigen binding site. The Fab' differs from the Fab in that it has a hinge region containing one or more cysteine residues at the C terminus of the heavy chain CH1 domain. The F(ab')₂ is formed when the cysteine residue in the hinge region of Fab' forms a disulfide bond. The Fv refers to the minimum antibody fragment containing only the heavy chain variable region and light chain variable region. In the heavy chain Fv (two-chain Fv), the heavy chain variable region and light chain variable region are connected by a non-covalent bond. In the single-chain Fv (single-chain Fv), the heavy chain variable region and light chain variable region are generally covalently linked through a peptide linker or are directly linked at the C-terminus, forming a dimer-like structure like double-chain Fv. The antigen binding fragment can be produced using proteolytic enzymes (for example, Fab can be obtained by restriction digestion of the entire antibody with papain, and F(ab')₂ fragment can be obtained by digestion with pepsin) or through genetic recombination technology, but is not limited thereto.

In the present invention, the term "humanized antibody" refers to an antibody that exhibits reduced immunogenicity or non-immunogenicity in humans. The humanized antibody may be manufactured by combining, for example, CDRs (complementarity determining regions) derived from entities other than humans (non-human species) with constant regions derived from human antibodies and FRs (framework regions) among variable regions derived from human antibodies. The humanized antibody can be produced by grafting the CDR of a non-human antibody between the FR sequences of a human antibody through the CDR-grafting method.

In the present invention, the term "FR (framework regions)" refers to the portion of the variable region of an immunoglobulin molecule other than the complementarity-determining region. The framework area has four framework areas (framework area 1, framework area 2, framework area 3, and framework area 4) in the light chain and heavy chain, respectively.

In the present invention, the term "human antibody" refers to an antibody of both light chain and heavy chain origin from humans. Depending on the difference in the constant region of the heavy chain, the human antibody includes IgG (including IgG1, IgG2, IgG3, and IgG4) having a γ chain heavy chain, IgM having a µ chain heavy chain, IgA (including IgA1 and IgA2) having an α chain heavy chain, IgD having a δ chain heavy chain, or IgE having an ε chain heavy chain. Also, in principle, the light chain includes one or more of a κ chain and a λ chain.

In the present invention, the term "chimeric antibody" refers to an antibody in which the variable region of the antibody is derived from an entity other than a human (non-human species) and the constant region is derived from a species other than the entity, such as a human.

The humanized antibody and chimeric antibody are described in more detail. When comparing mouse-derived antibodies and human antibodies, they have sequence similarities with human antibodies in the following order: mouse antibody - chimeric antibody - humanized antibody - human antibody. Therefore, compared to mouse antibodies or chimeric antibodies, humanized antibodies have a higher similarity to human antibodies and have the characteristic of low immunogenicity when administered to the human body.

In the present invention, the term "epitope" refers to a specific site on an antigen that an immunoglobulin, antibody, or antigen binding fragment thereof can specifically recognize and bind to. The epitope can be formed from continuous amino acids or from discontinuous amino acids juxtaposed by tertiary folding of the protein.

The humanized antibody of the present invention or an antigen binding fragment thereof comprises: a heavy chain variable region comprising FR-H1 having an amino acid sequence of SEQ ID NO: 1, FR-H2 having an amino acid sequence of SEQ ID NO: 2 or an amino acid sequence of SEQ ID NO: 3, FR-H3 having an amino acid sequence of SEQ ID NO: 4 and FR-H4 having an amino acid sequence of SEQ ID NO: 5; and a light chain variable region comprising FR-L1 having an amino acid sequence of SEQ ID NO: 6, FR-L2 having an amino acid sequence of SEQ ID NO: 7, FR-L3 having an amino acid sequence of SEQ ID NO: 8 and FR-L4 having an amino acid sequence of SEQ ID NO: 9, and specifically binds to TM4SF4(TransMembrane 4 Superfamily Member 4).

FR-H1 having the amino acid sequence of SEQ ID NO: 1 may be the FR1 sequence of the heavy chain variable region, referred to as `Hz2B7-1.0', `Hz2B7-2.0', `Hz2B7-3.0', `Hz2B7-4.0' in the present invention. FR-H2 having the amino acid sequence of SEQ ID NO: 2 may be the FR2 sequence of the heavy chain variable region, referred to as 'Hz2B7-1.0', 'Hz2B7-3.0', 'Hz2B7-4.0' in the present invention, and FR-H2 having the amino acid sequence of SEQ ID NO: 3 may be the FR2 sequence of the heavy chain variable region, referred to as 'Hz2B7-2.0' in the present invention. FR-H3 having the amino acid sequence of SEQ ID NO: 4 may be the FR3 sequence of the heavy chain variable region, referred to as `Hz2B7-1.0', 'Hz2B7-2.0', 'Hz2B7-3.0', 'Hz2B7-4.0' in the present invention. FR-H4 having the amino acid sequence of SEQ ID NO: 5 may be the FR4 sequence of the heavy chain variable region, referred to as `Hz2B7-1.0', `Hz2B7-2.0', `Hz2B7-3.0', `Hz2B7-4.0' in the present invention.

FR-L1 having the amino acid sequence of SEQ ID NO: 6 may be the FR1 sequence of the light chain variable region, referred to as 'Hz2B7-0.1', 'Hz2B7-0.2', 'Hz2B7-0.3' in the present invention. FR-L2 having the amino acid sequence of SEQ ID NO: 7 may be the FR2 sequence of the light chain variable region, referred to as 'Hz2B7-0.1', 'Hz2B7-0.2', 'Hz2B7-0.3' in the present invention. FR-L3 having the amino acid sequence of SEQ ID NO: 8 may be the FR3 sequence of the light chain variable region, referred to as `Hz2B7-0.1', 'Hz2B7-0.2', 'Hz2B7-0.3' in the present invention. FR-L4 having the amino acid sequence of SEQ ID NO: 9 may be the FR4 sequence of the light chain variable region, referred to as 'Hz2B7-0.1', 'Hz2B7-0.2', 'Hz2B7-0.3' in the present invention.

TM4SF4 is a type of tetraspanin protein, and is a protein known to be involved in apoptosis and differentiation in cancer cells, and cell invasion ability. In addition, TM4SF4 is known to promote the growth and metastasis of cancer stem cells, and can make tumors more malignant by strengthening the characteristics of cancer stem cells. The TM4SF4 protein may be a membrane protein present in the cell membrane, and a portion of TM4SF4 may be exposed to the outside of the cell. The exposed site may include two loop structures, and some amino acid sequences of the exposed extracellular sites of TM4SF4 may be epitopes that the humanized antibody or an antigen binding fragment of the present invention can specifically recognize and bind to. The epitope of TM4SF4 may include, for example, an amino acid sequence of 'TWGYPFHDGDYLNDE' (order from N-terminus to C-terminus).

The heavy chain variable region may further comprise at least one CDR selected from the group consisting of CDR-H1 having an amino acid sequence of SEQ ID NO: 10, CDR-H2 having an amino acid sequence of SEQ ID NO: 77 and CDR-H3 having an amino acid sequence of SEQ ID NO: 14. In particular, the CDR-H2 may have an amino acid sequence of SEQ ID NO: 78, and specifically, may have any one amino acid sequence selected from the group consisting of SEQ ID NO: 11, SEQ ID NO: 12 and SEQ ID NO: 13.

The light chain variable region may further comprise at least one CDR selected from the group consisting of CDR-L1 having an amino acid sequence of SEQ ID NO: 79, CDR-L2 having an amino acid sequence of SEQ ID NO: 18 and CDR-L3 having an amino acid sequence of SEQ ID NO: 80. In particular, the CDR-L1 may have any one amino acid sequence selected from the group consisting of SEQ ID NO: 15, SEQ ID NO: 16 and SEQ ID NO: 17, and the CDR-L3 may have an amino acid sequence of SEQ ID NO: 19.

The CDR-H1 having the amino acid sequence of SEQ ID NO: 10 may be the CDR1 sequence of the heavy chain variable region, referred to as 'Hz2B7-1.0', `Hz2B7-2.0', `Hz2B7-3.0', 'Hz2B7-4.0' in the present invention. The CDR-H2 having the amino acid sequence of SEQ ID NO: 11 may be the CDR2 sequence of the heavy chain variable region, referred to as 'Hz2B7-1.0', `Hz2B7-2.0' in the present invention, the CDR-H2 having the amino acid sequence of SEQ ID NO: 12 may be the CDR2 sequence of the heavy chain variable region, referred to as 'Hz2B7-3.0' in the present invention, and the CDR-H2 having the amino acid sequence of SEQ ID NO: 13 may be the CDR2 sequence of the heavy chain variable region, referred to as 'Hz2B7-4.0' in the present invention. The CDR-H3 having the amino acid sequence of SEQ ID NO: 14 may be the CDR3 sequence of the heavy chain variable region, referred to as 'Hz2B7-1.0', 'Hz2B7-2.0', `Hz2B7-3.0', `Hz2B7-4.0' in the present invention.

The CDR-L1 having the amino acid sequence of SEQ ID NO: 15 may be the CDR1 sequence of the light chain variable region, referred to as 'Hz2B7-0.1' in the present invention, the CDR-L1 having the amino acid sequence of SEQ ID NO: 16 may be the CDR1 sequence of the light chain variable region, referred to as 'Hz2B7-0.2' in the present invention, and the CDR-L1 having the amino acid sequence of SEQ ID NO: 17 may be the CDR1 sequence of the light chain variable region, referred to as 'Hz2B7-0.3' in the present invention. The CDR-L2 having the amino acid sequence of SEQ ID NO: 18 may be the CDR2 sequence of light chain variable region, referred to as 'Hz2B7-1.0', `Hz2B7-2.0', 'Hz2B7-0.3' in the present invention. The CDR-L3 having the amino acid sequence of SEQ ID NO: 19 may be the CDR3 sequence of the heavy chain variable region, referred to as 'Hz2B7-1.0', 'Hz2B7-2.0', 'Hz2B7-0.3' in the present invention.

The heavy chain variable region may have any one amino acid sequence selected from the group consisting of SEQ ID NO: 20, SEQ ID NO: 21, SEQ ID NO: 22 and SEQ ID NO: 23.

The light chain variable region may have any one amino acid sequence selected from the group consisting of SEQ ID NO: 24, SEQ ID NO: 25 and SEQ ID NO: 26.

The heavy chain variable region having the amino acid sequence of SEQ ID NO: 20 may be the sequence of the heavy chain variable region, referred to as `Hz2B7-1.0' in the present invention, the heavy chain variable region having the amino acid sequence of SEQ ID NO: 21 may be the sequence of the heavy chain variable region, referred to as 'Hz2B7-2.0' in the present invention, the heavy chain variable region having the amino acid sequence of SEQ ID NO: 22 may be the sequence of the heavy chain variable region, referred to as 'Hz2B7-3.0' in the present invention, and the heavy chain variable region having the amino acid sequence of SEQ ID NO: 23 may be the sequence of the heavy chain variable region, referred to as 'Hz2B7-4.0' in the present invention.

The light chain variable region having the amino acid sequence of SEQ ID NO: 24 may be the sequence of the light chain variable region, referred to as 'Hz2B7-0.1' in the present invention, the light chain variable region having the amino acid sequence of SEQ ID NO: 25 may be the sequence of the light chain variable region, referred to as 'Hz2B7-0.2' in the present invention, and the light chain variable region having the amino acid sequence of SEQ ID NO: 26 may be the sequence of the light chain variable region, referred to as 'Hz2B7-0.3' in the present invention.

The humanized antibody of the present invention or an antigen binding fragment thereof may include the heavy chain constant region and/or light chain constant region of an antibody derived from human. As long as the humanized antibody or an antigen binding fragment thereof does not inhibit the specific binding characteristics to TM4SF4, the heavy chain constant region and/or light chain constant region of the human-derived antibody can be used without limitation. For example, the heavy chain constant region may be a heavy chain constant region having the amino acid sequence of SEQ ID NO: 27, and the light chain constant region may be a light chain constant region having the amino acid sequence of SEQ ID NO: 28.

The amino acid sequences described above may include variants having different sequences due to deletion, insertion , substitution of amino acid residues, or combination thereof to the extent that they do not affect the structure, function, or activity of the polypeptide containing them. In addition, the amino acid sequences may include amino acids that have undergone common modifications known in the art. The amino acid modification may include, for example, phosphorylation, sulfation, acrylation, glycosylation, methylation, farnesylation, etc. The humanized antibody of the present invention or an antigen binding fragment thereof not only contains the amino acid sequences described above, but also includes those having a substantially identical amino acid sequence or a variant thereof. Having a substantially identical amino acid sequence may mean including an amino acid sequence having at least 90%, at least 91%, at least 92%, at least 93%, at least 94%, at least 95%, at least 96%, at least 97%, at least 98%, at least 99%, or at least 99.5% homology to the amino acid sequence described above, but is not limited thereto.

The humanized antibody of the present invention or an antigen binding fragment thereof may have an equilibrium dissociation constant(KD) of 3.0×10⁻⁸ M or less, for example, 2.9×10⁻⁸ M or less, 2.8×10⁻⁸ M or less, 2.7×10⁻⁸ M or less, 2.6×10⁻⁸ M or less or 2.5×10⁻⁸ M or less, and specifically 2.4×10⁻⁸ M or less.

According to a specific embodiment of the present invention, as a result of measuring and comparing the binding capacity for TM4SF4 through indirect ELISA using the humanized antibody of the present invention, it was confirmed that the humanized antibodies of the present invention exhibited significantly higher binding affinity, compared to a chimeric antibody (antibody combining the variable region of mouse-derived ECL-2B7 antibody and the constant region of human antibody) (Fig. 10A, 10B). In particular, among humanized antibodies using various combinations of light chain variable region and heavy chain variable region, the humanized antibody referred to as Hz2B7-1.2 showed the highest binding capacity. (Figs. 10, 11). In addition, the humanized antibody of the present invention did not show binding affinity in the indirect ELISA experiment performed on BSA, so it was confirmed that it is an antibody that specifically binds only to TM4SF4 (Fig. 10) .

According to another specific embodiment, as a result of FACS experiments using the humanized antibody of the present invention, it was confirmed that the humanized antibodies of the present invention had excellent binding capacity against Calu-3 and A549 cells, lung cancer cell lines that overexpress TM4SF4 on the cell surface (Fig. 12). Compared to the results of the FACS experiment using the chimeric antibody, the binding affinity to lung cancer cells was measured to be higher when the humanized antibody of the present invention was used, so, as in the indirect ELISA experiment, the humanized antibody called Hz2B7-1.2 was found to be the most effective (Figs. 10 to 12).

### 2. Technology for expressing TM4SF4 specific humanized antibody

Another aspect of the present invention provides a polynucleotide, an expression vector and a host cell that can be used to express and manufacture the humanized antibody or an antigen binding fragment thereof, and a method for producing thereof.

Since the description of the humanized antibody, its antigen binding fragment, TM4SF4, etc. is the same as that described in '1. Humanized antibody specifically binding to TM4SF4 and an antigen binding fragment thereof', the description is omitted to avoid repetitive explanation, and below, only the contents related to a polynucleotide, an expression vector, and a host cell are explained.

In the present invention, the term "polynucleotide" comprehensively includes DNA and RNA molecules, and nucleotides, which are the basic structural units of the polynucleotide, may include not only nucleotides that exist in nature, but also analogues with modified sugar or base sites.

The polynucleotide of the present invention includes a base sequence encoding a humanized antibody or an antigen binding fragment thereof.

Encoding the humanized antibody or an antigen binding fragment thereof means that the polynucleotide encodes genetic information that can synthesize a protein having the amino acid sequence of the humanized antibody of the present invention or an antigen binding fragment through typical protein expression processes such as transcription and translation. In this case, not only a protein having an amino acid sequence completely identical to the humanized antibody or an antigen binding fragment thereof, but also a protein having an amino acid sequence substantially identical to the above protein, as described above, or a polynucleotide encoding a protein having the same and/or similar activity as the above protein may be included in the scope of the present invention.

The polynucleotide may contain an optimized base sequence depending on the type of organism to be introduced and expressed and the expression system such as transcription and translation of the organism.

Specifically, the polynucleotide may include at least one base sequence selected from the group consisting of SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55 and SEQ ID NO: 56.

The base sequence of SEQ ID NO: 29 may encode the amino acid sequence of SEQ ID NO: 1, the base sequence of SEQ ID NO: 30 may encode the amino acid sequence of SEQ ID NO: 2, the base sequence of SEQ ID NO: 31 may encode the amino acid sequence of SEQ ID NO: 3, the base sequence of SEQ ID NO: 32 may encode the amino acid sequence of SEQ ID NO: 4, the base sequence of SEQ ID NO: 33 may encode the amino acid sequence of SEQ ID NO: 5, the base sequence of SEQ ID NO: 34 may encode the amino acid sequence of SEQ ID NO: 6, the base sequence of SEQ ID NO: 35 may encode the amino acid sequence of SEQ ID NO: 7, the base sequence of SEQ ID NO: 36 may encode the amino acid sequence of SEQ ID NO: 8, the base sequence of SEQ ID NO: 37 may encode the amino acid sequence of SEQ ID NO: 9, the base sequence of SEQ ID NO: 38 may encode the amino acid sequence of SEQ ID NO: 10, the base sequence of SEQ ID NO: 39 may encode the amino acid sequence of SEQ ID NO: 11, the base sequence of SEQ ID NO: 40 may encode the amino acid sequence of SEQ ID NO: 12, the base sequence of SEQ ID NO: 41 may encode the amino acid sequence of SEQ ID NO: 13, the base sequence of SEQ ID NO: 42 may encode the amino acid sequence of SEQ ID NO: 14, the base sequence of SEQ ID NO: 43 may encode the amino acid sequence of SEQ ID NO: 15, the base sequence of SEQ ID NO: 44 may encode the amino acid sequence of SEQ ID NO: 16, the base sequence of SEQ ID NO: 45 may encode the amino acid sequence of SEQ ID NO: 17, the base sequence of SEQ ID NO: 46 may encode the amino acid sequence of SEQ ID NO: 18, the base sequence of SEQ ID NO: 47 may encode the amino acid sequence of SEQ ID NO: 19, the base sequence of SEQ ID NO: 48 may encode the amino acid sequence of SEQ ID NO: 20, the base sequence of SEQ ID NO: 49 may encode the amino acid sequence of SEQ ID NO: 21, the base sequence of SEQ ID NO: 50 may encode the amino acid sequence of SEQ ID NO: 22, the base sequence of SEQ ID NO: 51 may encode the amino acid sequence of SEQ ID NO: 23, the base sequence of SEQ ID NO: 52 may encode the amino acid sequence of SEQ ID NO: 24, the base sequence of SEQ ID NO: 53 may encode the amino acid sequence of SEQ ID NO: 25, the base sequence of SEQ ID NO: 54 may encode the amino acid sequence of SEQ ID NO: 26, the base sequence of SEQ ID NO: 55 may encode the amino acid sequence of SEQ ID NO: 27, and the base sequence of SEQ ID NO: 56 may encode the amino acid sequence of SEQ ID NO: 28.

The polynucleotide of the present invention may contain a base sequence that is substantially identical to the base sequences listed above. The substantially identical base sequence refers to a case where, for example, the same amino acid can be synthesized when transcribed and translated, and may be a base sequence having at least 90%, at least 91%, at least 92%, at least 93%, at least 94%, at least 95%, at least 96%, at least 97%, at least 98%, at least 99%, or at least 99.5% homology to the base sequences listed above, but is not limited thereto.

The expression vector of the present invention contains the above polynucleotide.

In the present invention, the term "expression vector" refers to a means for expressing a specific gene in a host cell. Specifically, the expression vector includes plasmid vector; Cosmid vector; and viral vectors such as bacteriophage vectors, adenovirus vectors, retrovirus vectors, and adeno-associated virus vectors, but are not limited thereto.

In addition to the base sequence encoding the humanized antibody or an antigen binding fragment, the expression vector may further include regulatory sequences such as a promoter and terminator, and the base sequence encoding the humanized antibody or an antigen binding fragment thereof may be operatively linked to a promoter. The operably linked means a functional linkage between a regulatory sequence (e.g., a promoter, a signal sequence, an array of transcriptional regulator binding sites, etc.) and another base sequence, whereby the regulatory sequence can regulate transcription and/or translation of the other base sequence.

The expression vector system of the present invention can be constructed through various methods known in the art.

The expression vector can be constructed using a prokaryotic cell or a eukaryotic cell as a host.

For example, when the expression vector uses a prokaryotic cell as a host, it generally includes a strong promoter(e.g., tac promoter, lac promoter, lacUV5 promoter, lpp promoter, pLλ, promoter, pRλ promoter, rac5 promoter, amp promoter, recA promoter, SP6 promoter, trp promoter and T7 promoter, etc) capable of advancing transcription, a ribosome binding site for translation initiation, and a transcription/translation termination sequence. For example, when the expression vector uses a prokaryotic cell as a host, it generally includes a strong promoter capable of driving transcription, a ribosome binding site for translation initiation, and a transcription/translation termination sequence. When E. coli(for example, HB101, BL21, DH5α, etc.) is used as a host cell, the promoter and operator region(Yanofsky, C, J Bacteriol, (1984) 158:1018-1024) of the E coli tryptophan biosynthesis pathway and the left-handed promoter of phage λ(pLλ promoter, Herskowitz, I and Hagen, D, Ann Rev Genet, (1980) 14:399-445) can be used as control regions. When Bacillus bacteria are used as host cells, the promoter of the toxin protein gene of Bacillus thuringiensis(Appl Environ Microbiol (1998) 64:3932-3938; Mol Gen Genet (1996) 250:734-741) or any promoter that can be expressed in Bacillus bacteria can be used as a control region. The expression vector can be created by manipulating plasmids(For example, pCL, pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phages (e.g., λgt4·λB, λ-Charon, λΔz1, and M13, etc.) or viruses (e.g., SV40, etc.) that are often used in the art.

When the expression vector uses a eukaryotic cell as a host, a promoter derived from the genome of a mammalian cell (For example, metallothionein promoter, β-actin promoter, human hemoglobin promoter and human muscle creatine promoter.) or a promoter derived from a mammalian virus(For example, adenovirus late promoter, vaccinia virus 75K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter and Rous sarcoma virus (RSV) promoter) can be used, and may generally have a polyadenylation sequence as a transcription termination sequence. The expression vector may have a CMV promoter.

Additionally, the expression vector can be fused with other sequences to facilitate purification of antibodies expressed therefrom. Sequences to be fused include, for example, glutathione S-transferase (Pharmacia, USA), maltose binding protein (NEB, USA), FLAG (IBI, USA), and 6x His (hexahistidine; Quiagen, USA). In addition, since the protein expressed by the expression vector of the present invention is a humanized antibody or an antigen binding fragment thereof, considering its characteristics, the expressed protein can be easily purified through a protein A column, etc. without additional sequences for purification.

The expression vector contains an antibiotic resistance gene commonly used in the art as a selection marker, and may include, for example, a resistance gene to ampicillin, gentamicin, carbenicillin, chloramphenicol, streptomycin, kanamycin, geneticin, neomycin, and tetracycline.

The expression vector may be a vector system in which the light chain and heavy chain are simultaneously expressed in one vector, or a system in which the light chain and heavy chain are expressed in separate vectors. In the latter case, the two vectors can be introduced into the host cell through, for example, co-transformation or targeted transformation. Co-transformation is a method of simultaneously introducing each vector DNA encoding a light chain and a heavy chain into a host cell and then selecting cells that express both the light chain and the heavy chain. Targeted transformation is a method of selecting cells transformed with a vector containing a light chain(or heavy chain), transforming the selected cells again with a vector containing a heavy chain (or light chain), and finally selecting cells that express both the light chain and the heavy chain.

The host cell of the present invention includes the above expression vector.

As the host cell, any host cell known in the art can be used as long as it can stably and continuously clone and express the expression vector of the present invention. The host cell is a prokaryotic host cell, for example, Escherichia coli, Bacillus strains such as Bacillus subtilis and Bacillus thuringiensis, Streptomyces, Pseudomonas such as Pseudomonas putida, Proteus mirabilis, or Staphylococcus such as Staphylococcus carnosus, but is not limited thereto.

When the host cell is a eukaryotic host cell, fungi such as Aspergillus species, yeast such as Pichia pastoris, Saccharomyces cerevisiae, Schizosaccharomyces and Neurospora crassa, other lower eukaryotes, higher eukaryotes such as insect-derived cells, and cells derived from plants or mammals can be used. The host cell may be COS7 cell (monkey kidney cells), NSO cell, SP2/0, Chinese hamster ovary(CHO) cell, W138, baby hamster kidney(BHK) cell, MDCK, baby hamster kidney, HuT 78 cell or 293 cell, but is not limited thereto.

Transformation and/or transfection into the host cell can be performed using any method for introducing nucleic acids into an organism, cell, tissue or organ, and can be performed by selecting an appropriate standard technique depending on the host cell, as known in the art. Specifically, electroporation, protoplast fusion, calcium phosphate (CaPO₄) precipitation, calcium chloride (CaCl₂) precipitation, stirring using silicon carbide fiber, agrobacteria-mediated transformation, PEG, dextran sulfate, lipofectamine and drying/inhibition-mediated transformation methods, etc., but is not limited thereto.

The method of producing the humanized antibody of the present invention or an antigen binding fragment thereof includes the step of culturing the host cell.

The method of producing the humanized antibody or an antigen binding fragment thereof may further include the step of expressing the humanized antibody or an antigen binding fragment thereof in the host cell.

Cultivation of the host cells can be performed according to appropriate media and culture conditions known in the art. This culture process can be easily adjusted and used by those skilled in the art according to the selected strain. Cell culture is divided into suspension culture and adherent culture depending on the growth method of the cells, and batch, fed-batch, and continuous culture depending on the culture method. The medium used for culture must appropriately meet the requirements of the specific strain.

In animal cell culture, the medium contains various carbon sources, nitrogen sources, and trace element components. Examples of the carbon sources that can be used include carbohydrates such as glucose, sucrose, lactose, fructose, maltose, starch and cellulose, fats such as soybean oil, sunflower oil, castor oil and coconut oil, fatty acids such as palmitic acid, stearic acid and linoleic acid, alcohols such as glycerol and ethanol, and organic acids such as acetic acid, and these carbon sources may be used alone or in combination.

Examples of the nitrogen sources include organic nitrogen sources such as peptone, yeast extract, broth, malt extract, corn steep liquor (CSL) and soybean meal, and inorganic nitrogen sources such as urea, ammonium sulfate, ammonium chloride, ammonium phosphate, ammonium carbonate, and ammonium nitrate, and these nitrogen sources can be used alone or in combination.

The medium may include potassium dihydrogen phosphate, dipotassium hydrogen phosphate, and corresponding sodium-containing salts as a phosphorous source. Additionally, it may contain metal salts such as magnesium sulfate or iron sulfate. In addition, amino acids, vitamins, and appropriate precursors may be included.

In the culturing step, the pH of the culture can be adjusted by adding compounds such as ammonium hydroxide, potassium hydroxide, ammonia, phosphoric acid, and sulfuric acid to the culture in an appropriate manner. Additionally, during culturing, foam generation can be suppressed by using an antifoaming agent such as fatty acid polyglycol ester. Additionally, in order to maintain the aerobic state of the culture, oxygen or oxygen-containing gas (e.g., air) is injected into the culture. The temperature of the culture can usually be between 20°C and 45°C or between 25°C and 40°C.

The production method may further include the step of recovering the humanized antibody or an antigen binding fragment thereof expressed in the host cell. The humanized antibody or an antigen binding fragment thereof obtained by culturing the transformed host cell may be used in an unpurified state, or can be further purified to high purity using various conventional methods, such as dialysis, salt precipitation, and chromatography. When using chromatography, the type and order of columns can be selected from ion exchange chromatography, size exclusion chromatography, affinity chromatography, etc. depending on the characteristics of the antibody, culture method, etc.

### 3. Use of the humanized antibody of the present invention for detection of TM4SF4

Another aspect of the present invention provides the use of the humanized antibody or an antigen binding fragment thereof for detecting TM4SF4. Specifically, the present invention provides a composition for detecting TM4SF4, a kit for detecting TM4SF4, and a method for detecting TM4SF4.

The description of the humanized antibody, its antigen binding fragment, TM4SF4, etc. is the same as that described in '1. Humanized antibody specifically binding to TM4SF4 and an antigen binding fragment thereof', so the description is omitted to avoid repeated explanation.

The composition for detecting TM4SF4 of the present invention includes the humanized antibody or an antigen binding fragment thereof, and the kit for detecting TM4SF4 of the present invention includes the composition for detecting TM4SF4.

Additionally, the method for detecting TM4SF4 of the present invention includes the step of contacting the humanized antibody or an antigen binding fragment thereof with a sample to be detected that is expected to contain TM4SF4.

The composition for detecting TM4SF4 and the kit containing thereof can effectively detect TM4SF4 by forming an antigen-antibody complex by contacting the humanized antibody or an antigen binding fragment of the present invention that specifically binds to TM4SF4 to the sample to be detected.

The antigen-antibody complex refers to a combination of TM4SF4 and an antibody that recognizes it, to identify tumors or cancer cells expressing TM4SF4 in a sample.

Quantification of TM4SF4 antigen using the composition for detecting TM4SF4, the kit containing thereof, or the humanized antibody or an antigen binding fragment thereof can be performed by confirming the formation of an antigen-antibody complex. Confirmation of the formation of the antigen-antibody complex can be performed by enzyme-linked Enzyme-linked immunosorbent assay (ELISA), Western Blotting, Immunofluorescence, Immunohistochemistry staining, Flow cytometry, Immunocytochemistry, radioimmunoassay (RIA), Immunoprecipitation Assay, Immunodiffusion assay, Complement Fixation Assay, Protein Chip, etc., but is not limited thereto. 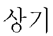 The enzyme-linked immunosorbent assay (ELISA) includes various ELISA methods, for example, Direct ELISA using a labeled antibody that recognizes an antigen attached to a solid support, Indirect ELISA using a labeled secondary antibody that recognizes a capture antibody in a complex of an antibody that recognizes an antigen attached to a solid support, Direct sandwich ELISA using another labeled antibody that recognizes the antigen in a complex of antibody and antigen attached to a solid support, Indirect sandwich ELISA, which involves reacting a complex of antibody and antigen attached to a solid support with another antibody that recognizes the antigen, and then using a labeled secondary antibody that recognizes this antibody, etc..

Labels that enable the formation of antigen-antibody complexes to be measured qualitatively or quantitatively include, but are not limited to, enzymes, fluorescent substances, ligands, luminescent substances, microparticles, redox molecules, and radioisotopes. The enzymes include β-glucuronidase, β-D-glucosidase, β-D-galactosidase, urease, peroxidase, alkaline phosphatase, acetylcholinesterase, glucose oxidase, hexokinase and GDPase, RNase, glucose oxidase and luciferase, phosphofructokinase, phosphoenolpyruvate carboxylase, aspartate aminotransferase, phosphophenolpyruvate decarboxylase, β-ratamase, etc., but is not limited thereto.

### 4. Use of humanized antibody of the present invention for prevention or treatment of cancer, use for inhibiting cancer stem cell growth and use for assisting radiation anticancer treatment

Another aspect of the present invention provides a pharmaceutical composition for preventing or treating cancer containing the humanized antibody or an antigen binding fragment thereof, a composition for inhibiting the growth of cancer stem cells, and a composition for assisting radiation anticancer treatment.

The description of the humanized antibody, an antigen binding fragment thereof, TM4SF4, etc. is the same as that described in '1. Humanized antibody specifically binding to TM4SF4 and an antigen binding fragment thereof', so the description is omitted to avoid repeated explanation.

However, the humanized antibody or an antigen binding fragment thereof of the present invention may include a CDR sequence that is identical to the CDR sequence of the 2B7 antibody or a partial sequence thereof modified. For the characteristics and effects of the 2B7 antibody, refer to Korean Patent Application No. 10-2020-0168467.

The pharmaceutical composition for preventing or treating cancer of the present invention includes the humanized antibody or an antigen binding fragment thereof.

The humanized antibody or an antigen binding fragment thereof can bind to TM4SF4 with high affinity, and TM4SF4 is known to be over-expressed on the surface of cancer cells. Accordingly, the humanized antibody or an antigen binding fragment thereof can be used to target cancer cells.

The composition can be used alone as the humanized antibody or an antigen binding fragment thereof or in combination with a conventional pharmaceutically acceptable carrier for the treatment, prevention and diagnosis of hyperproliferative diseases such as cancer.

The cancer may specifically be lung cancer, stomach cancer, colorectal cancer, colon cancer, triple-negative breast cancer, glioblastoma, pancreatic cancer, head and neck cancer, breast cancer, ovarian cancer, kidney cancer, bladder cancer, prostate cancer, endometrial cancer, salivary gland cancer, or thyroid cancer. More specifically, it may be lung cancer, breast cancer, liver cancer, kidney cancer, stomach cancer, pancreatic cancer, and brain cancer, but is not limited thereto. In the present invention, the cancer may be, but is not limited to, cancer caused by over-expression, amplification, mutation, or activation of TM4SF4. In other words, the composition containing the humanized antibody or binding fragment thereof of the present invention has a proliferation inhibitory effect on all carcinomas regardless of abnormal expression or mutation of TM4SF4, so the medicinal use of the present invention is not limited by the expression pattern or mutation of TM4SF4.

The composition may be in the form of a pharmaceutical composition, a quasi-drug composition, or a health food composition.

The composition for preventing or treating cancer of the present invention may further comprise a pharmaceutically acceptable carrier. The meaning of 'pharmaceutically acceptable' is that it does not inhibit the activity of the active ingredient and does not have toxicity beyond what the application (prescription) target can adapt to, and the 'carrier' is defined as a compound that facilitates the addition of a compound into cells or tissues.

The pharmaceutical composition of the present invention can be administered alone or mixed with any convenient carrier, and the dosage form may be a single dose or repeated dose form. The pharmaceutical composition may be a solid preparation or a liquid preparation. The solid preparation may include, but are not limited to, powders, granules, tablets, capsules, suppositories, etc. The solid preparation may include, but is not limited to, a carrier, flavoring agent, binder, preservative, disintegrant, lubricant, filler, etc. The liquid preparation include, but are not limited to, solutions such as water and propylene glycol solutions, suspensions, and emulsions, and may be prepared by adding appropriate colorants, flavoring agents, stabilizers, viscosifiers, etc. For example, powders can be prepared by simply mixing a tri-hydroxy derivative of a polyunsaturated fatty acid, which is the active ingredient of the present invention, with a suitable pharmaceutically acceptable carrier such as lactose, starch, or microcrystalline cellulose. The granules can be prepared using a wet granulation method using a solvent such as water, ethanol, or isopropanol, or a dry granulation method using compression force after mixing the tri-hydroxy derivative of the polyunsaturated fatty acid of the present invention, a suitable pharmaceutically acceptable carrier, and a suitable pharmaceutically acceptable binder such as polyvinylpyrrolidone and hydroxypropyl cellulose. Additionally, tablets can be prepared by mixing the granules with a suitable pharmaceutically acceptable lubricant such as magnesium stearate and then compressing the mixture into tablets using a tablet press.

The pharmaceutical composition may be administered as oral agents, injections (e.g., intramuscular injection, intraperitoneal injection, intravenous injection, infusion, subcutaneous injection, implant), inhalation agent, intranasal administration, vaginal agent, rectal administration agent, sublingual agent, transdermal agent, topical agent, etc., depending on the disease to be treated and the condition of the individual, but is not limited thereto. Depending on the route of administration, it may be formulated into an appropriate dosage unit formulation containing commonly used non-toxic pharmaceutically acceptable carriers, excipients, and vehicles.

The pharmaceutical composition may be administered in a daily dose of about 0.0001 mg/kg to about 10 g/kg, and may be administered in a daily dosage of about 0.001 mg/kg to about 1 g/kg. However, the dosage may vary depending on the degree of purification of the mixture, the patient's condition (age, gender, weight, etc.), and the severity of the condition being treated. If necessary, for convenience, the total daily dose may be administered in divided doses several times throughout the day.

The composition for inhibiting the growth of cancer stem cells of the present invention includes the humanized antibody or an antigen binding fragment thereof.

The cancer stem cell (CSC) refers to an undifferentiated cell that has the ability to differentiate into various cancer cells. Cancer stem cells exist in approximately 1-2% of malignant tumor tissues and have self-replication and pluripotency, which are characteristics of normal stem cells. Due to abnormalities in self-regulatory function, the number of cells increases due to cell division activation and they differentiate into malignant tumor cells. Due to these characteristics of cancer stem cells, general cancer cells are removed through anticancer treatment, but cancer stem cells survive, and it is known that cancer recurrence and metastasis occur due to some of the surviving cancer stem cells.

Specifically, the cancer stem cell of the present invention may be a cancer cell that over-expresses the ALDH1 (aldehyde dehydrogenase 1) protein, which is one of the markers of cancer stem cells, or has positive protein activity.

The humanized antibody or an antigen binding fragment thereof of the present invention can selectively inhibit cancer stem cells, and in particular, can obtain excellent anticancer effects by killing cancer cell groups containing cancer stem cells that are highly resistant to anticancer treatment. The humanized antibody or an antigen binding fragment thereof can inhibit the growth of cancer stem cells by reducing the self-renewal ability, invasion ability, and migration ability of cancer stem cells.

The antibody or antigen binding fragment thereof can be used to prevent or treat cancers with cancer stem cell characteristics, but is not limited thereto. cancers with cancer stem cell characteristics show resistance to existing anti-cancer treatments and have a poor prognosis, so treatment different from existing anti-cancer treatments must be applied. For example, even if the patient has the same type of cancer, if the type of cancer has a high proportion of cancer stem cells, the patient will not be able to obtain cancer treatment effects from existing anticancer treatments such as anticancer drugs or radiation therapy. Therefore, even if it is the same type of cancer, if the proportion of cancer stem cells in the cells at the cancer lesion site is high, it is very important to apply a new treatment method that is different from the existing anticancer treatment.

The cancers with cancer stem cell characteristics may be cancers with a high proportion of cancer stem cells in the cell group constituting the cancer. Considering that the proportion of cancer stem cells among general cancer cells is about 1% or more and less than 5%, for example, cases where the proportion of cancer stem cells in the cell group constituting cancer is 5% or more, 10% or more, 30% or more, 50% or more, or 70% or more can be defined as cancers with cancer stem cell characteristics. As described above, it can be characterized by resistance to existing anti-cancer treatments and a poor prognosis for anti-cancer treatment. Specifically, in the present invention, the cancers with cancer stem cell characteristics may be cancers that overexpress ALDH1. The cancer that over-expresses ALDH1 may be a cancer in which the proportion of cancer stem cells that express ALDH1 or are positive for its activity is relatively higher than that of general cancer.

Specifically, the cancer over-expressing ALDH1 may be any one or more selected from the group consisting of lung cancer, breast cancer, liver cancer, kidney cancer, stomach cancer, pancreatic cancer, and brain cancer, but is not limited thereto.

The prevention or treatment of cancer refers to preventing or treating cancer chemoresistance during or after cancer treatment, cancer recurrence, or cancer metastasis by reducing the regenerative ability, growth ability, invasion ability, or migration ability of cancer stem cells.

The composition for assisting radiation anticancer treatment of the present invention includes the humanized antibody or an antigen binding fragment thereof.

The composition includes the humanized antibody or an antigen binding fragment thereof as an active ingredient to improve the radiation sensitivity of cancer-related cells.

The cancer-related cells are cells that constitute cancer, and may have the characteristics of having an irregular shape, proliferating indefinitely, and having weak cohesion with surrounding cells compared to normal cells. Specifically, the cancer-related cells may be cancer cells or cancer stem cells, and may specifically be cancer stem cells.

The cancer stem cell may be an undifferentiated cell with the ability to differentiate into various cancer cells, and may specifically be a cancer cell that expresses ALDH1 or is activity positive. In the present invention, the cancer stem cells may have the characteristics that cell proliferation is not inhibited by radiation, self-renewal ability is not reduced, and migration and invasion abilities are not inhibited.

In addition, the cancer-related cells may have low sensitivity to radiation, that is, high resistance to radiation therapy, and may have substantially no sensitivity to radiation, making anticancer treatment by radiation impossible.

The anti-cancer treatment may inhibit proliferation of cancer-related cells, inhibit metastasis and invasion, and induce cell death through radiation, surgery, chemotherapy, etc. In the present invention, the anti-cancer treatment may be administration of the humanized antibody or an antigen binding fragment thereof in combination with radiation. In this way, when the humanized antibody or antigen binding fragment is administered in combination with radiation, the radiation sensitivity of cancer-related cells is improved by the antibody or antigen binding fragment, thereby maximizing the effect of anticancer treatment by radiation, and further preventing recurrence and metastasis of cancer.

Hereinafter, the present invention will be described in detail by Examples.

However, the following Example specifically illustrates the present invention, and the content of the present invention is not limited by the following Examples.

### [Example and Comparative Example]

### [Example 1]

### Design of Humanized antibody specifically binding to TM4SF4 and preparation of expression vector

A humanized antibody that can specifically bind to the TM4SF4 protein with high affinity was prepared. For this purpose, a novel humanized antibody that has specific binding to TM4SF4 and shows low immunogenicity when administered to the human body was prepared, using the amino acid sequence of the CDR (complementarity determining regions) of the variable region among the antibody regions to form specific binding to TM4SF4, and the amino acid sequence of the constant region and the amino acid sequence of the FR (framework regions) of the variable region of human antibodies.

Specifically, the CDR sequence of the humanized antibody of the present invention is the CDR sequence of a mouse-derived antibody capable of specifically binding to TM4SF4, and the following sequence was used: CDR-H1(SEQ ID NO: 10), CDR-H2(SEQ ID NO: 11) and CDR-H3(SEQ ID NO: 14) of a heavy chain variable region, and CDR-L1(SEQ ID NO: 15), CDR-L2 (SEQ ID NO: 18) and CDR-L3 (SEQ ID NO: 19) of a light chain variable region. In addition, as FR sequences, FR-H1(SEQ ID NO: 1), FR-H2(SEQ ID NO: 2), FR-H3(SEQ ID NO: 4) and FR-H4(SEQ ID NO: 5) of a heavy chain variable region, and FR-L1(SEQ ID NO: 6), FR-L2(SEQ ID NO: 7), FR-L3(SEQ ID NO: 8) and FR-L4(SEQ ID NO: 9) were used. The CDR sequences used the CDR sequence of the ECL-2B7 antibody, a mouse-derived TM4SF4 antibody, and the FR sequences used the FR sequence of the 3QRG antibody, a human antibody. The 3QRG antibody was selected from a group of human antibody candidates similar to the heavy chain and light chain amino acid sequences of the ECL-2B7 antibody using the BLASTP analysis program of the National Center for Biotechnology Information (NCBI). The humanized antibody of the present invention was prepared by grafting the CDR sequence to the FR sequence using a CDR-grafting method. First, based on the amino acid sequence of the CDR sequence obtained by adding a signal peptide sequence to the front of the CDR sequences (SEQ ID NO: 10, SEQ ID NO: 11 and SEQ ID NO: 14) of the heavy chain variable region among the CDR sequences, the sequence was converted into a base sequence encoding it using a reverse translate program (https://www.bioinformatics.org/sms2/rev trans.html). Then, using recombinant PCR, the base sequence was linked with the FR sequences (SEQ ID NO: 1, SEQ ID NO: 2, SEQ ID NO: 4, and SEQ ID NO: 5) to produce a gene encoding the heavy chain variable region of the humanized antibody of the present invention. The gene of about 450 bp in length was divided into three sections. As shown in Figure 1, fragments of the heavy chain variable region gene corresponding to T1(164 bp), T2(167 bp), and T3 (157 bp) were synthesized using ECL-2B7-S-H-5' and ECL-2B7-S-H-3' primers for T1, ECL-2B7-HC-1-5' and ECL-2B7-HC-1-3' primers for T2, and ECL-2B7-HC-2-5' and ECL-2B7-HC-2-3' primers for T3. Then, the T1 and T2 fragments were linked together through recombinant PCR to synthesize a fragment of 310 bp, and the linked fragment of T1 and T2 was again linked to the T3 fragment using Ch57-H.C-5' and ECL-2B7-WH-3' primers, to synthesize a heavy chain variable region (Hz2B7-1.0) gene of the humanized antibody of the present invention of about 450 bp with EcoRI and ApaI restriction enzyme cleavage sites at both ends. The heavy chain variable region was manufactured to be arranged in the following order: FR-H1, CDR-H1, FR-H2, CDR-H2, FR-H3, CDR-H3, and FR-H4, and this was confirmed on an agarose gel (Fig. 2). The primer sequences used in this process are shown in Table 1 below. In addition, the gene of the light chain variable region (Hz2B7-0.1) of the humanized antibody of the present invention with a length of about 450 bp was synthesized by requesting a gene synthesis company (Bionics, Korea) to be arranged in the following order: FR-L1(SEQ ID NO: 6), CDR-L1(SEQ ID NO: 15), FR-L2(SEQ ID NO: 7), CDR-L2(SEQ ID NO: 18), FR-L3(SEQ ID NO: 8), CDR-L3(SEQ ID NO: 19), FR-L4(SEQ ID NO: 9), and this was confirmed on an agarose gel (Fig. 3). Then, the heavy chain variable region gene was cloned into the EcoRI-ApaI site of the pdCMV-dhfr vector using T4 ligase (NEB, USA), and the light chain variable region gene was cloned into the HindIII-BsiWI site using T4 ligase(Fig. 8) to prepare vector pdCMV-dhfrC-Hz2B7-1.1 encoding the humanized antibody (Hz2B7-1.1) of the present invention.

**[Table 1]**

| Name | Sequence (5' ->3') | SEQ ID NO |
|---|---|---|
| ECL-2B7-S-H-5' primer | GAC GAA TTC ACT CTA ACC ATG GAA TGG AGC TGG GTC TTT CTC TTC TTC CTG TCA GTA ACT ACA GGT GTC CAC TCC GAG ATC ACC CTG AA | SEQ ID NO: 57 |
| ECL-2B7-S-H-3' primer | CTC AGG CTG AAG CCG CTG AAG GTG CAG GTC AGG GTC AGG GTC TGG GTG GGC TTC ACC AGG GTG GGG CCG CTC TCC TTC AGG GTG ATC TCG GAG | SEQ ID NO: 58 |
| ECL-2B7-S-H-5' primer | CAG CGG CTT CAG CCT GAG CAC TTA TGG TAT AGG AGT AAG CTG GAT CAG GCA GCC CCC CGG CAA GGC CCT GGA GTG GCT GGC CCA CAT TTG | SEQ ID NO: 59 |
| ECL-2B7-S-H-5' primer | CCT GGT TCT TGC TGG TGT CCT TGG TGA TGG TCA | SEQ ID NO: 60 |
| | GCC TGC TCT TCA GGG CTG TGT TAT AGT ACT TAT TAT CAT TCC ACC AAA TGT GGG CCA GCC AC | |
| ECL-2B7-S-H-5' primer | ACA CCA GCA AGA ACC AGG TGG TGC TGA CCA TGA CCA ACA TGG ACC CCG TGG ACA CCG CCA CCT ACT ACT GCG CCA GGA AGG AGG GCA GCT | SEQ ID NO: 61 |
| ECL-2B7-S-H-5' primer | GGG CCC TTG GTG GAG GCG CTG CTC ACG GTC ACC AGG GTG CCC TGG CCC CAG TAA GCA AAG GGG GCC GAG CTG CCC TCC TTC CTG G | SEQ ID NO: 62 |
| Ch57.H.C-5' primer | GAC GAA TTC ACT CTA ACC AT | SEQ ID NO: 63 |
| ECL-2B7-WH-3' primer | TTG GGC CCT TGG TGG AGG CGC TGC T | SEQ ID NO: 64 |

### [Example 2] Design of variant antibody of the humanized antibody of the present invention and preparation of expression vector

### [2-1] Interaction analysis and molecular modeling between the humanized antibody of the present invention and the epitope

In order to prepare a variant antibody in which some of the amino acid sequences of the humanized antibody of the present invention designed through Example 1 were modified, first, the interaction between the humanized antibody and the target epitope of TM4SF4 was confirmed. In general, the binding force of antigen-antibody can be qualitatively predicted through docking simulation. Since the X-ray structure of the humanized antibody in Example 1 is unknown, its structure was predicted through homology modeling, and based on this, the binding force between the antibody and the epitope was confirmed. For this, version 8.2 of the MODELLER program was used, and Mouse chimeric antibody X836, which has high similarity between the humanized antibody in Example 1 and the amino acid sequence, was used as a template for homology modeling using BLAST. Among the 235 amino acids of the X836 antibody, 176 amino acids were identical to the humanized antibody of Example 1, showing an amino acid sequence similarity of about 75%, and based on this, the three-dimensional structure of the humanized antibody of Example 1 could be predicted (Fig. 4).

Since the epitope of the antigen, TM4SF4 protein, is a short polypeptide chain, docking simulation was performed in the CDR region of the humanized antibody in Example 1 above, considering it as an organic compound, and the binding free energy and binding mode were calculated. Specifically, calculations were performed using the 15-mer consisting of the amino acid sequence Thr-Trp-Gly-Tyr-Pro-Phe-His-Asp-Gly-Asp-Tyr-Leu-Asn-Asp-Glu from positions 126 to 140 of the TM4SF4 protein as a simplified model for the entire antigen. The binding free energy of the epitope for the humanized antibody was calculated to be -11.6 kcal/mol, and when converted to Ki value, it was calculated to be about 3 nM. This means that a very strong antigen-antibody binding will be formed between the humanized antibody of the present invention and the epitope of TM4SF4. As a docking program, a modified version of AutoDock version 4.2.6 developed by the Scripps Research Institute in the United States was used, and some terms of the protein-ligand binding free energy function were improved to enhance accuracy. Specifically, the parameters contained in the electrostatic interaction term, hydrogen bond energy term, and hydration energy term of the ligand were optimized more accurately and used in the docking simulation. In the electrostatic interaction section between the antibody and the epitope, the dielectric constant value of the antibody molecule was directly calculated and used, and in the hydrogen bond energy term, the accuracy of the calculation was enhanced by expressing it as a product of the Morse function to reflect the interdependence of angle and distance. In the ligand hydration energy section, accuracy was improved by increasing the number of parameters to 69 and optimizing them using a genetic algorithm. The weighting factor values for the van der Waals bond, hydrogen bond, electrostatic interaction, entropy, and hydration energy terms were 0.1485, 0.0656, 0.1146, 0.3113, and 0.1711, respectively, and the values given in the existing AutoDock program were used as is.

Through the structural analysis results of the complex between the humanized antibody and epitope of Example 1 obtained through the above docking simulation, the amino acid region that plays an important role in binding was identified. Among the amino acids of the humanized antibody Hz2B7-1.1, five amino acids, arginine at position 99 (Arg, R) in the light chain, asparagine (Asn, N) at position 58 in the heavy chain, tyrosine (Tyr, Y) at position 60 in the heavy chain, glycine (Gly. G) at position 102 of the heavy chain, and serine (Ser, S) at position 103 of the heavy chain, belong to the CDR region and are expected to play a key role in the function of the antibody by forming hydrogen bonds with the epitope (Figs. 4 and 5). The van der Waals bond was found to be relatively weak, and among the hydrophobic amino acids, only tyrosine at position 38 of the light chain and tyrosine at position 98 of the light chain were observed at the interface of the antibody-epitope complex (Figs. 4 and 5).

Amino acids that directly interact with the epitope through hydrogen bonds or van der Waals bonds are maintained as they play an important role in the function of the antibody, and in order to increase the binding affinity to the epitope, it is necessary to substitute amino acids that have weak binding affinity or are located at a long distance. For example, in Figs. 4 and 5, when asparagine (Asn, N) at position 31 corresponding to CDR1 of the light chain variable region of the humanized antibody Hz2B7-1.1, which is the part where the phenylalanine and tryptophan residues of the epitope bind, is replaced with a more hydrophobic aromatic amino acid, it was predicted that the binding force would increase as the van der Waals bond between and antibody was strengthened. In addition, tryptophan (Trp, W) at position 55, corresponding to CDR2 of the heavy chain variable region of the humanized antibody Hz2B7-1.1, is located adjacent to aspartic acid of the epitope, so when replaced with an amino acid that can form a stronger hydrogen bond, it was predicted that binding force would increase. Therefore, position 31, corresponding to CDR1 of the light chain variable region, and position 55, corresponding to CDR2 of the heavy chain variable region, are important positions that can increase epitope and affinity (Figs. 4 and 5). In addition, three amino acids, including threonine (Thr, T) at position 100 corresponding to CDR3 of the light chain variable region, tryptophan at position 54 corresponding to CDR2 of the heavy chain variable region, and asparagine at position 56 corresponding to CDR2 of the heavy chain variable region, are also located relatively close to the epitope. Therefore, it is expected that replacing these with amino acids with different chemical properties can increase the efficacy of the antibody by inducing a new hydrogen bond or van der Waals bond with the epitope. Therefore, positions 100, corresponding to CDR3 of the light chain variable region, and positions 54 and 56, corresponding to CDR2 of the heavy chain variable region, are also other important positions that can increase epitope and affinity (Figs. 4 and 5).

### [2-2] Design of variant humanized antibody amino acid sequence and preparation of vector

Through the docking simulation analysis of Example 2-1, by substituting alanine (Ala, A) at position 46 of the humanized antibody heavy chain variable region of Example 1 with valine (Val, V), the heavy chain variable region Hz2B7-2.0 (HC A46V) was designed with the amino acid sequence of SEQ ID NO: 3 among the humanized antibody sequences in Example 1 as FR-H2 (FIG. 5).

In addition, by substituting tryptophan (Trp, W) at position 55 of the humanized antibody heavy chain variable region of Example 1 with serine (Ser, S) or tyrosine (Tyr, Y), respectively, the heavy chain variable region Hz2B7-3.0(HC W55S) and the heavy chain variable region Hz2B7-4.0(HC W55Y) were designed with the amino acid sequence of SEQ ID NO: 12 or SEQ ID NO: 13, respectively, among the humanized antibody sequences in Example 1 as CDR-H2 (FIG. 5). For this, h2B7-H.C-W55S-5' and h2B7-H.C-W55S-3' primers, and h2B7-H.C-W55Y-5' and h2B7-H.C-W55Y-3' primers were synthesized, and for in-fusion cloning, recombinant PCR was performed using h2B7-H.C-SLIC-3' primer and h2B7-H.C-SLIC-3' primer (Fig. 6A). The sequence of the synthesized gene was cloned into the pdCMV-dhfr-h2B7 vector cut with EcoR1 and ApaI using a kit (EZ-Fusion^{™} HT Cloning Kit, Enzymomics, Korea) (Fig. 8). After confirming the mutation through base sequence analysis, pdCMV-dhfr-Hz2B7-3.0 and pdCMV-dhfr-Hz2B7-4.0, which can express the variant humanized antibody, were prepared.

In addition, by substituting asparagine (Asn, N) at position 31 of the humanized antibody light chain variable region of Example 1 with phenylalanine (Phe, F) or valine (Val, V), respectively, the light chain variable region Hz2B7-0.2(LC N31F) and the light chain variable region Hz2B7-0.3(LC N31V) were designed with the amino acid sequence of SEQ ID NO: 16 or SEQ ID NO: 17, respectively, among the humanized antibody sequences in Example 1 as CDR-L1, and confirmed on agarose gel (FIG. 5). For this, h2B7-L.C-N31F-5' and h2B7-L.C-N31F-3' primers, and h2B7-L.C-N31V-5' and h2B7-L.C-N31V-3' primers were synthesized, and for in-fusion cloning, recombinant PCR was performed using h2B7-L.C-SLIC-3' primer and h2B7-L.C-SLIC-3' primer (Fig. 6B). The sequence of the synthesized gene was cloned into the pdCMV-dhfr-h2B7-1.1 vector cut with HindIII and BsiWI using a kit (EZ-Fusion^{™} HT Cloning Kit, Enzymomics, Korea) (Fig. 8). After confirming the mutation through base sequence analysis, pdCMV-dhfr-Hz2B7-0.2 and pdCMV-dhfr-Hz2B7-0.3, which can express the variant humanized antibody, were prepared.

The primer sequences used in this process are shown in Table 2 below.

**[Table 2]**

| Name | Sequence(5'->3') | SEQ ID NO |
|---|---|---|
| h2B7-H.C-W55Y-5' primer | TTT GGT ATA ATG ATA ATA AGT A | SEQ ID NO: 65 |
| h2B7-H.C-W55Y-3' primer | TCA TTA TAC CAA ATG TGG GCC A | SEQ ID NO: 66 |
| h2B7-H.C-W55S-5' primer | TTT GGT CGA ATG ATA ATA AGT A | SEQ ID NO: 67 |
| h2B7-H.C-W55S-3' primer | TCA TTC GAC CAA ATG TGG GCC A | SEQ ID NO: 68 |
| h2B7-H.C-SLIC-5' primer | GCC AGT GTG CTG GAA TTC ACT CTA ACC | SEQ ID NO: 69 |
| h2B7-H.C-SLIC-3' primer | AAG ACC GAT GGG CCC TTG GTG GAG | SEQ ID NO: 70 |
| h2B7-L.C-N31V-5' primer | TTT TAG TAA GTA GCA ATC AA | SEQ ID NO: 71 |
| h2B7-L.C-N31V-3' primer | CTA CTT ACT AAA AGG CTC TGA C | SEQ ID NO: 72 |
| h2B7-L.C-N31F-5' primer | TTT TAT TCA GTA GCA ATC AA | SEQ ID NO: 73 |
| h2B7-L.C-N31F-3' primer | CTA CTG AAT AAA AGG CTC TGA C | SEQ ID NO: 74 |
| h2B7-L.C-SLIC 5' primer | ATA GGG AGA CCC AAG CTT CGG CAC GAG CAG A | SEQ ID NO: 75 |
| h2B7-L.C-SLIC 3' primer | TGG TGC AGC CAC CGT ACG CTT GAT CTC CA | SEQ ID NO: 76 |

### [Comparative Example] Design of chimeric antibody targeting TM4SF4 and preparation of expression vector

The humanized antibodies of Examples 1 and 2 above were manufactured using the CDR sequence of a mouse antibody as the CDR sequence among the heavy chain/light chain variable regions of the antibody and used the sequence of a human antibody as the FR sequence among the constant region and variable region of the antibody. Therefore, in order to compare with the humanized antibody of the present invention, a chimeric antibody was prepared in which the entire heavy chain/light chain variable region was derived from a mouse antibody and the constant region was derived from a human antibody. In other words, the chimeric antibody can be viewed as an antibody that falls between a mouse antibody and a humanized antibody in terms of similarity to a human antibody.

First, PCR was performed on the heavy chain and light chain genes of the mouse-derived ECL-2B7 antibody, and DNA of approximately 400 bp and 390 bp was separated using a gel extraction kit (FAVORGEN, Taiwan). In order to synthesize the signal sequence of the heavy chain gene, PCR was performed using the pdCMV-dhfr vector as a template and the 5'-signal-EcoR1 (5'-GAC GAA TTC ACT CTA ACC ATG GAA TGG A) primer and ECL-2B7-S-H-3' (CTT CAC CTC GGA GTG GAC ACC TGT AGT TA-3') primer (Fig. 7A). And to amplify the heavy chain variable region, PCR was performed using the DNA of the heavy chain variable region as a template and the 2B7-S-H-5' (5'-GTC CAC TCC GAG GTG AAG CTG GAG GAG TC) primer and the ECL-2B7-HC-Chi-3' (TTG GGC CCT TGG TGG AGG CTG CAG AGA CAG TGA CCA G-3') primer(Fig. 7A). Then, to link the heavy chain signal sequence with the heavy chain variable region gene, recombinant PCR was performed using the 5'-signal-EcoR1 primer and the ECL-2B7-HC-Chi-3' primer. Through this, the gene in which the heavy chain variable region and the heavy chain signal sequence were connected was obtained and confirmed by running it on a 1% agarose gel, and DNA corresponding to about 450 bp was isolated using a DNA isolation kit (FavorPrep GEL^{™} PCR Purification Kit, Farvorgen, Taiwan) (Fig. 7A). The heavy chain variable region gene linked to the signal sequence was treated with EcoRI and ApaI and then isolated using the FavorPrep GEL^{™} PCR Purification Kit (Fig. 7B). The heavy chain variable region gene thus obtained was cloned into the EcoRI and ApaI sites of the pdCMV-dhfr vector containing the heavy chain constant region (IgG1) gene of a human antibody using T4 DNA ligase (NEB, USA). (Fig. 8). Likewise, in the case of light chain genes, in order to synthesize the signal sequence, PCR was performed using the pdCMV-dhfr-ch57 vector as a template and the ch57-LC-whole 5' (5'-CTG CAA AGC TTC GGC ACG AGC A) primer and ECL-2B7-S-L-3' (CAC AAT ATC TCC TTC AAC ACC AGA CAA CC-3') primer, and to amplify the light chain variable region gene, PCR was performed using the ECL-2B7-S-L-5' (5'-GTT GAA GGA GAT ATT GTG ATG ACC CAG TCT) primer and ECL-2B7-LC-chi-3' (CCA CCG TAC GTT TGA TTT CCA GCT T-3') primer (Fig. 7A). To link the signal sequence with the light chain variable region, recombinant PCR was performed using ch57-LC-whole 5' primer and ECL-2B7-LC-chi-3' primer, confirmed by running it on a 1% agarose gel, and then isolated in the same manner as the heavy chain variable region gene (Fig. 7A). The light chain variable region gene was isolated by treatment with HindIII and BsiwI. Then, by cloning the gene into the HindIII and BisWI sites of the pdCMV-dhfr-chi 2B7-HC vector, which contains the light chain constant region (Ck) gene of a human antibody and into which the previously prepared heavy chain gene is inserted (Fig. 7C), a vector capable of expressing the chimeric antibody was prepared (pdCMV-dhfr-chi2B7, Fig. 8). The vector was transformed into E. coli DH5α using the RbCl2 method, and then E. coli with a size of approximately 450bp were selected. These E. coli were cultured overnight in 5 ml of LB medium containing 50 *µg*/ml of ampicillin. Then the plasmid DNA was isolated using a kit for plasmid DNA isolation (DNA-spin^{™} Plasmid DNA purification kit, INTRON, Korea) and the base sequence was confirmed (Bionics, Korea). As a result, it was confirmed that the base sequence of the cDNA isolated from the transformant was identical to the variable region gene of the ECL-2B7 antibody and was correctly linked to the heavy chain and light chain constant region genes of the human antibody.

### [Experimental Example 1]

### Expression and purification of humanized antibody and chimeric antibody

Antibody proteins were expressed from the humanized antibody vector of the present invention prepared through Examples 1 and 2 above and the chimeric antibody vector of the Comparative Example above and purified.

First, to express and purify the chimeric antibody (`Chi2B7'), 1 × 10⁷ HEK293T cells (human embryonic kidney cell line) were cultured in 20 ml of DMEM medium (Biowest, France) using a 150 mm culture dish. The cultured cells were mixed with 50 µg of the pdCMV-dhfr-chi2B7 expression vector and 75 µl of polyethyleneimine (1 mg/ml), then mixed with 500 ul of transfection optimization medium and evenly sprinkled on the cell culture medium. The next day, the supernatant was collected, filled with new medium, the chimeric antibody expressed from the cell line was collected, and the supernatant was placed in a column packed with beads (Protein G agarose beads, Amicogen, Korea), allowing the antibody to bind to the beads. Then, the beads were washed with PBS (pH8.0), and the antibody was eluted from the beads using 10 ml of 0.1M glycine (pH2.8) and 1M Tris-HCl (pH9.0) to purify the Chi2B7 chimeric antibody. This was confirmed through SDS-PAGE and Coomassie blue staining (Fig. 9A). In addition, whether the purified chimeric antibody has the constant region of the human antibody was detected by Western blotting using goat antibodies (goat-α-hIgG-gamma chain-HRP, Invitrogen, USA, and goat-α-hIgG-kappa chain-HRP, Bethyl , USA) as a secondary antibody. As a result, it was confirmed that the chimeric antibody was an antibody containing the constant region of a human antibody (Fig. 9B).

Additionally, the humanized antibodies of Examples 1 and 2 above were expressed and purified. Through Examples 1 and 2 above, four types of heavy chain variable regions and three types of light chain variable regions of the humanized antibody of the present invention were manufactured, and various types of humanized antibodies can be manufactured through their combination. Accordingly, the expression vectors of Examples 1 and 2 were introduced into HEK293T cells, and the humanized antibody was expressed using the same method as the chimeric antibody, and was separated and purified using Protein G beads and columns.

To confirm the purity of the purified chimeric antibody, the humanized antibody of the present invention, and the mouse-derived 2B7 antibody, 10% SDS-PAGE was performed. Among a total of 10 humanized antibodies, 6 representative types (Hz2B7-1.1, Hz2B7-1.2, Hz2B7-1.3, Hz2B7-2.1, Hz2B7-3.2, Hz2B7-4.3) were analyzed. After SDS-PAGE, Coomassie blue staining and Western blotting analysis were performed using 1 µg of each antibody (Fig. 9). As a result of staining with Coomassie blue staining solution (PageBlue staining solution, Thermo Scientific, USA), the heavy chain and light chain of each antibody were confirmed at approximately 55 kDa and 25 kD positions (Fig. 9A). This means that both the chimeric antibody and the humanized antibody are IgG-type antibodies and have partial sequences of human antibodies. For Western blotting, goat antibodies (goat-α-hIgG-gamma chain-HRP, Invitrogen, USA, and goat-α-hIgG-kappa chain-HRP, Bethyl, USA) were used as secondary antibodies, and the band was detected using an ECL kit (Western Bright ECL kit, advansta, USA). As a result, the ECL-2B7 antibody was not detected through Western blotting because it was a mouse antibody. In the case of other antibodies (chimeric antibody and humanized antibody), the heavy chain and light chain of each antibody were detected in the same manner as the SDS-PAGE results, confirming that they had human antibody sequences, and the purity of the antibody (Fig. 9B).

### [Experimental Example 2]

### Comparison of TM4SF4 antigen binding capacity of humanized antibodies through ELISA

Binding capacity for TM4SF4 was confirmed through indirect ELISA using the humanized antibody and chimeric antibody of the present invention.

Specifically, the humanized antibody was manufactured in 10 types of Hz2B7-1.1, Hz2B7-1.2, Hz2B7-1.3, Hz2B7-2.1, Hz2B7-3.1, Hz2B7-3.2, Hz2B7-3.3, Hz2B7-4.1, Hz2B7-4.2 and Hz2B7-4.3, by combining 4 heavy chain variable region variants and 3 light chain variable region variants designed through Examples 1 and 2 above. For this, 100 µℓ of TM4SF4-BSA (1 µg/mℓ) or BSA solution (1 µg/mℓ) dissolved in coating buffer(100 mM carbonate/bicarbonate coating buffer, pH 9.6) was added to the bottom of each well of a 96-well plate and adsorbed at 4°C for more than 16 hours. Next, the coated antigen was washed three times with washing buffer(wash buffer, 0.05% PBS-T, Tween-20), then 200 µℓ of block buffer (blocking buffer, 5% skim milk in wash buffer) was added to the bottom of each well where the antigen was not adsorbed, reacted at 4°C for more than 16 hours, and then washed again three times with washing buffer. Then, the 10 combinations of the humanized antibodies and the chimeric antibodies purified through Experimental Example 1 were prepared by diluting them to various concentrations using block buffer, and then 100 µℓ of each concentration was added to each well and reacted for 2 hours at room temperature. After washing again three times, 1 mg/ml antibody (a-human IgG-Kappa chain-HRP, Bethyl, USA) for detection was diluted 10,000-fold using block buffer solution, and 100 µℓ was added to each well and incubated at room temperature for 1 hour. After the reaction was completed, the wells were washed three times with washing buffer, 100 µl of substrate solution(OPD solution, 4 µℓ 30% H₂O₂, 100 µℓ OPD stock (40 mg/mℓ), adjusted to 10 mℓ with phosphate citrate buffer) was added to each well, and the reaction was performed at room temperature. Then, 50 ul of 2.5M H₂SO₄ was added to terminate the reaction. Finally, to measure the activity of the enzyme bound to the solid phase, the absorbance was measured at 490 nm using an ELISA reader (Fig. 10).

As a result, all 10 humanized antibodies of the present invention did not bind to BSA, and appeared to bind with high specificity to TM4SF4-BSA antigen (Fig. 10). Therefore, it was confirmed that the humanized antibody of the present invention not only has binding ability to TM4SF4, but also has the characteristic of binding specifically to it. Additionally, when comparing the affinity for TM4SF4-BSA, all 10 humanized antibodies showed significantly higher affinity for TM4SF4 than the chimeric antibody Chi2B7. In addition, 10 humanized antibodies showed different binding affinities, and among them, the Hz2B7-1.2 humanized antibody showed a clearly increased binding affinity compared to Hz2B7-1.1. Therefore, it was confirmed that the binding ability to TM4SF4 was the best (Figs. 10, A and B).

### [Experimental Example 3]

### Comparison of sensitivity of anti-TM4SF4 humanized monoclonal antibody through SPR analysis

Surface plasmon resonance (SPR) analysis was performed to compare the binding capacity of the constructed anti-TM4SF4 humanized monoclonal antibodies to human TM4SF4-peptide (hTM4SF4 aa 126-140) antigen.

There are a total of 5 types of humanized antibodies that are analytes, and as shown in Table 3, one initial mutation type (HzE2B7-1.1), two light chain mutation types (HzE2B7-1.2(LC N31F), HzE2B7-1.3(LC N31V)), and two heavy chain/light chain mutation types (HzE2B7-4.3(HC W55Y, LC N31V), HzE2B7-3.2(HC W55S, LC N31F)) were analyzed. A synthetic peptide in which biotin is bound to the amino acid sequence from positions 126 to 140 of the human TM4SF4 protein(Biotin-**GSAGGS**TWGYPFHDGDYLNDE, **GSAGGS**: space sequence between Biotin and TWGYPFHDGDYLNDE, TM4SF4 aa126-140) was used as a ligand.

**[Table 3]**

| Antibody | Heavy Chain mutation | Light Chain mutation | KD (M) |
|---|---|---|---|
| HzE2B7-1.1(origin) | - | - | 2.442 × 10⁻⁸ |
| HzE2B7-4.3(HC W55Y, LC N31V) | W55Y | N31V | 8.165 × 10⁻⁸ |
| HzE2B7-1.2(LC N31F) | - | N31F | 6.030 × 10⁻⁹ |
| HzE2B7-1.3(LC N31V) | - | N31V | 3.542 × 10⁻⁸ |
| HzE2B7-3.2(HC W55S, LC N31F) | W55S | N31F | 1.119 × 10⁻⁷ |

First, a streptavidin-conjugated sensor chip (Series S Senser Chip SA, Cytiva) was inserted into Biacore T200 (Cytiva), and activation buffer (1M NaCl, 50mM NaOH) was flowed for 30 seconds, followed by HBS-EP buffer (Cytiva) to induce activation and stabilization of the sensor chip surface. By flowing the ligand (Biotin-hTM4SF4 aa 126-140) at a concentration of 1nM to 128nM to the stable sensor chip, the ligand was immobilized on the surface of the sensor chip, and by flowing the regeneration solution (Regeneration buffer, 20mM NaOH), a streptavidin conjugated sensor chip with a stable biotin-peptide ligand was constructed. The analyte antibody for affinity analysis was prepared from 512 nmol/L to a concentration of 16 nmol/L by sequentially diluting it by 1/2. The analyze antibodies of each concentration were flowed through the constructed ligand-bound streptavidin conjugated sensor chip at a rate of 30 ul/min for 480 seconds to induce binding, and then HBS-EP buffer solution was flowed at the same time and speed, and the degree to which the analyte antibody was dissociated from the ligand peptide was obtained in the form of a response unit. The association rate (Ka), dissociation rate (Kd), and equilibrium dissociation constant (KD, Kd/Ka) values were calculated using Biacore T200 evaluation software (Cytiva) by excluding the ligand-unbound sensor chip response values obtained in the same manner. As a calculation standard, the mass transfer fitting model for the interaction between the ligand peptide and the analyte antibody was analyzed using 1:1 binding. Based on the equilibrium dissociation constant (KD) shown in Figure 11, the binding ability of anti-TM4SF4 humanized monoclonal antibodies to human TM4SF4-peptide (hTM4SF4 aa 126-140) antigen was lowest at 6.03×10⁻⁹ M for HzE2B7-1.2(LC N31F) antibody, 2.442×10⁻⁸ M for HzE2B7-1.1, 3.542 ×10⁻⁸ M for HzE2B7-1.3(LC N31V), 8.165 ×10⁻⁸ M for HzE2B7-4.3(HC W55Y LC N31V), and 1.119 ×10⁻⁷ M for HzE2B7-3.2(HC W55S LC N31F) (Table 3). It is more desirable for the equilibrium dissociation constant (KD) of a humanized antibody to be low, and for general commercially licensed humanized antibodies, the value is usually 10-8 M or less, and the lower limit is not particularly limited. Therefore, the KD of the initially designed humanized antibody (HzE2B7-1.1) already had a useful KD of 2.442×10₋₈ M. Based on the information obtained through the docking model, the binding ability of the HzE2B7-1.2(LC N31F) antibody, in which the 31st asparagine (N) of the light chain was replaced with phenylalanine (F), to the antigen increased by about 4 times compared to the originally designed HzE2B7-1.1, and the equilibrium dissociation constant (KD) value has reached 6.03×10⁻⁹ M, indicating that it is an antibody with higher commercial value.

### [Experimental Example 4]

### Comparison of cancer cell binding capacity of humanized antibodies through FACS

Through Experimental Example 2, it was confirmed that the humanized antibody of the present invention binds specifically to TM4SF4 with high affinity. Therefore, cancer cells that overexpress TM4SF4 were treated with the humanized antibody of the present invention to confirm binding.

Specifically, lung cancer cell lines Calu-3 and A549 cell lines were cultured in DMEM medium (Biowest, France) using 10% fetal bovine serum (WelGene). And the liver cancer cell lines Huh7, SNU-387, and SNU-449 cell lines were cultured in RPMI-1640 medium (Biowest) using 10% fetal bovine serum. Afterwards, the cells that had grown to about 80% were separated by treating with an enzyme (TrypLE^{™} Express Enzyme, Gibco^{™}), washed with PBS (pH 7.4) and then fixed with 4% paraformaldehyde (PFA) at 4°C for 15 minutes. After washing twice with PBA (0.1% BSA in PBS), 5 × 10⁵ cells for each antibody were treated with PBA contains the mouse mouse-derived 2B7 antibody (10 *µ*g/mℓ), the chimeric antibody Chi2B7 (10 *µ*g/mℓ), the humanized antibodies of the present invention, Hz2B7-1.1 (10 *µ*g/mℓ), Hz2B7-1.2 (10 *µ*g/mℓ) and Hz2B7-1.3 (10 *µ*g/mℓ), and mouse and human isotype control IgG1 antibody (10 *µ*g/mℓ), respectively, and reacted at 4°C for 1 hour. After reaction, washing twice with PBA, in the group containing the mouse-derived 2B7 antibody and the mouse isotype IgG (2 *µ*g/mℓ), Invitrogen), α-mouse IgG-FITC (2 *µ*g/mℓ) antibody, which is conjugated to FITC fluorescence and is specific for mouse IgG, was added. And in the group containing the human isotype IgG, the chimeric antibody, and the humanized antibody, α-Human IgG-FITC (2 *µ*g/mℓ, Invitrogen) antibody, which is specific for human IgG and is bound to FITC fluorescence, was added and reacted at 4°C for 30 minutes. After the reaction, the cells were washed with PBA and suspended in 500 *µℓ* of PBA, and then the antibodies were confirmed to have specificity for the antigen using a flow cytometer (FACS Calibur, BD, USA).

As a result, it was confirmed that, like the mouse-derived 2B7 antibody, the humanized antibody of the present invention and the chimeric antibody of Comparative Example specifically bound to Calu-3 and A549 lung cancer cells expressing TM4SF4 on the surface. In addition, although it did not bind to the surface of human primary hepatocyte (hPH), it was confirmed to bind specifically to liver cancer cell lines Huh7, SNU-387, and SNU-449 liver cancer cells. Among them, in the case of the humanized antibody Hz2B7-1.2 of the present invention, as a result of analysis, it was confirmed that it had a significantly high binding capacity, especially in the A549 cell line. (Fig. 12).

### [Experimental Example 5]

### Production of cell lines capable of producing the humanized antibody of the present invention with high productivity

To prepare a cell line capable of producing the humanized antibody of the present invention, the DG44 cell line, which is defective in the gene encoding dihydrofolate reductase among CHO cells (Chinese ovary hamster cells), was used. The DG44 cells were cultured in IMDM medium (Welgene, Korea) with the addition of H.T. (hypoxanthine-thymidine, Sigma, Germany) and Dialyed FBS (Gibco, USA), and the cell lines were detached with trypsin-EDTA (welgene, Korea), and then distributed at 1×10⁶ cells/well in a 6-well plate. In order to transfect the cells with expression vectors encoding Hz2B7-1.1 and Hz2B7-1.2 (LC N31F) among the humanized antibodies of the present invention, 15 *µ*g of pdCMV-dhfr-hz2B7-1.1 linearized by treatment with PvuI restriction enzyme(Enzynomics, Korea) was prepared by mixing well in TOM (transfection optimized medium) medium, reacted with 15 *µℓ* of lipofectamine 2000 (Invitrogen, USA) in TOM medium and then mixed with medium containing the vector and reacted for 20 minutes. Then, transformation was performed by dropping the reacted solution onto the DG44 cells.

The cells into which the gene encoding the humanized antibody of the present invention was introduced were screened for neomycin resistance in a medium containing G418 at a concentration of 100 ug/ml, and the supernatant of the cells in which colonies were formed was obtained. Production of antibody was confirmed by performing sandwich ELISA using the humanized antibody of the present invention contained in the supernatant. Among each transformant, five clones showing high OD values in ELISA results and excellent antibody expression were selected and treated with MTX (methotrexate), and then the antibody gene of the present invention was amplified. The antibody gene was amplified for 1 week by treating with MTX at a concentration of 0.02 µM. Next, antibody production was measured through ELISA, and five clones with increased production were selected again, and the genes were amplified a second time by increasing MTX to a concentration of 0.08 µM.

Accordingly, a total of five clones, 3A12, H12, 4A12, 4B12, and 4H12, were selected as cell lines that highly produce Hz2B7-1.1 humanized antibody. In addition, 4A9, 4A12, 4B9, 4C7, and 4H12 clones were selected as cell lines that highly produced Hz2B7-1.2 antibody (LC N31F), and a total of 7 clones were selected, including 7A8 and 6G10 clones obtained through additional transformation (Fig. 13). Cell stocks of the 12 clones were made and cultured in IMDM medium with 10% Dialyzed FBS. After the cells grew to about 50%, they were treated with MTX at a concentration of 0.08 µM every two days to amplify antibody genes. As a result of treatment and culture with MTX for about 30 days, it was possible to obtain a cell line resistant to MTX at a concentration of 0.08µM. The 4H12 clone was selected as a cell line with high expression of Hz2B7-1.1 antibody, and the 4A12 clone was selected as a cell line with high expression of Hz2B7-1.2 antibody.

In order to select single clones of the selected clones that survived the 0.08 µM concentration of MTX, 3×10⁴ cells were dispensed onto a 100 mm dish. After about 10 days, a stock was made using each colony formed and stored, and the remaining cells were checked for an increase in antibody production through sandwich ELISA. Specifically, 100 *µℓ* of α-human IgG-Fc specific antibody (2 *µ*g/mℓ) dissolved in coating buffer (100 mM carbonate/bicarbonate coating buffer, pH 9.6) was added to the bottom of each well of a 96-well plate, and adsorption was carried out at 4°C for more than 16 hours. After washing and blocking, the 4H12 clone (Hz2B7-1.1-4H12), 4A12 clone (Hz2B7-1.2-4A12), and Each single clone with an amplified antibody gene selected at a MTX concentration of 0.08µM was cultured in a CO2 incubator at 37°C for 24 hours, and then the culture medium was collected. The culture medium and human IgG isotype control antibody were prepared by diluting them to various concentrations, and then 100 ul of each concentration was added to each well and reacted at room temperature for 2 hours. Then, α-human IgG-Kappa chain-HRP (1 mg/ml, Bethyl, USA) was diluted 10,000-fold using blocking buffer, and 100 µl was added to each well and reacted at room temperature for 1 hour. After the reaction was completed, 100 *µℓ* of the substrate OPD solution (4 *µ*ℓ of 30% H2O2, 100 *µℓ* OPD stock (30% H₂O₂ 4 *µ*ℓ, 100 *µ*ℓ OPD stock (40 *µ*g/mℓ), adjusted to 10 mℓ with phosphate citrate buffer) was added to each well, reacted at room temperature for 10 minutes, and then 50 *µℓ* of 2.5 M H₂SO₄ was added to terminate the reaction.

As a result of ELISA, among the clones of Hz2B7-1.1-4H12-0.08 and Hz2B7-1.2-4A12-0.08 whose antibody genes were amplified by MTX, those clones that showed higher absorbance than the clones before amplification by MTX were identified. And antibody concentration was calculated through quantitative antibody production analysis (Figs. 14 and 15). As a result, in the case of the Hz2B7-1.1-4H12 clone, the antibody production of cells before the antibody gene was amplified by MTX was about 2.8 *µ*g/10⁶ cell/24hr, but the antibody production of the Hz2B7-1.1-4H12-0.08-#1 clone, whose antibody gene was amplified by MTX, increased approximately 3.8 times to 10.8 *µ*g/10⁶cell/24hr. In the case of the Hz2B7-1.2-4A12 clone, the antibody production before amplification was 2.5 *µ*g/10⁶ cell/24hr, but in the case of the Hz2B7-1.2-4A12-0.08 clone, which was amplified with an MTX concentration of 0.08M, the #9 clone showed an antibody production of approximately 14.2 *µ*g/10⁶ cell/24hr, and the #20 clone showed an antibody production of 7.9 *µ*g/10⁶ cell/24hr, showing an antibody production that was approximately 5.7-fold and 3.1-fold increased, respectively, compared to the clone in which the gene was not amplified. It can be seen that antibody productivity has increased by up to 5.7 times (Fig. 15).

Next, a serum stability experiment was conducted to measure the stability of the antibody in the blood. The mouse antibody 2B7 purified from a mouse hybridoma cell line and the humanized antibodies Hz2B7-1.1 and Hz2B7-1.2 purified from a CHO cell line were each cultured in 60% human serum (Sigma-Aldrich) at 37°C for up to 4 days. Antigen binding capacity at 0, 3, 24, 48, 72, and 96 hours was measured using indirect ELISA. Specifically, 0.5 ug/ml of TM4SF4-BSA antigen was adsorbed at 4°C for more than 16 hours using a coating buffer solution (100mM carbonate/bicarbonate coating buffer, pH 9.6), followed by washing and blocking processes. Afterwards, an ELISA process was performed in the same manner as before using the antibody cultured in human serum as the primary antibody to confirm whether antigen binding capacity was maintained over time. After measuring the absorbance, the antigen binding capacity at 0 hours was assumed to be 100% and the antigen binding capacity was compared. As a result, it was confirmed that mouse antibody 2B7 had equivalent antigen binding capacity in human serum up to 96 hours. (Fig. 16A). In addition, the humanized antibodies Hz2B7-1.1 and Hz2B7-1.2, whose antibody sequence and structure were changed, were confirmed to maintain a certain antigen binding capacity without being degraded in human serum for up to 4 days. (Fig. 16B and 16C).

Therefore, by specifically amplifying the antibody gene using MTX in CHO-DG44 cells transformed with the humanized antibody gene of the present invention, the Hz2B7-1.1-4H12-0.08-#1, Hz2B7-1.2-4A12-0.08-#9 cell line could be established as a cell line producing the antibody of the present invention.

In the above, the present invention has been described in detail only with respect to the described embodiments, but it is obvious to those skilled in the art that various changes and modifications are possible within the technical scope of the present invention, and it is natural that such changes and modifications fall within the scope of the appended claims.

## Claims

1. A humanized antibody or an antigen binding fragment thereof, which comprises:
a heavy chain variable region comprising FR-H1 having an amino acid sequence of SEQ ID NO: 1, FR-H2 having an amino acid sequence of SEQ ID NO: 2 or an amino acid sequence of SEQ ID NO: 3, FR-H3 having an amino acid sequence of SEQ ID NO: 4 and FR-H4 having an amino acid sequence of SEQ ID NO: 5; and
a light chain variable region comprising FR-L1 having an amino acid sequence of SEQ ID NO: 6, FR-L2 having an amino acid sequence of SEQ ID NO: 7, FR-L3 having an amino acid sequence of SEQ ID NO: 8 and FR-L4 having an amino acid sequence of SEQ ID NO: 9,
and specifically binds to TM4SF4(TransMembrane 4 Superfamily Member 4).

2. The humanized antibody or an antigen binding fragment thereof according to claim 1, wherein the heavy chain variable region further comprises at least one CDR selected from the group consisting of CDR-H1 having an amino acid sequence of SEQ ID NO: 10, CDR-H2 having an amino acid sequence of SEQ ID NO: 77 and CDR-H3 having an amino acid sequence of SEQ ID NO: 14, and
the light chain variable region further comprises at least one CDR selected from the group consisting of CDR-L1 having an amino acid sequence of SEQ ID NO: 79, CDR-L2 having an amino acid sequence of SEQ ID NO: 18 and CDR-L3 having an amino acid sequence of SEQ ID NO: 80.

3. The humanized antibody or an antigen binding fragment thereof according to claim 2, wherein the CDR-H2 has any one amino acid sequence selected from the group consisting of SEQ ID NO: 11, SEQ ID NO: 12 and SEQ ID NO: 13,
the CDR-L1 has any one amino acid sequence selected from the group consisting of SEQ ID NO: 15, SEQ ID NO: 16 and SEQ ID NO: 17, or
the CDR-L3 has an amino acid sequence of SEQ ID NO: 19.

4. The humanized antibody or an antigen binding fragment thereof according to claim 3, wherein the heavy chain variable region further comprises CDR-H1 having an amino acid sequence of SEQ ID NO: 10, CDR-H2 having an amino acid sequence of SEQ ID NO: 11 and CDR-H3 having an amino acid sequence of SEQ ID NO: 14, and
the light chain variable region further comprises CDR-L1 having an amino acid sequence of SEQ ID NO: 16, CDR-L2 having an amino acid sequence of SEQ ID NO: 18 and CDR-L3 having an amino acid sequence of SEQ ID NO: 19.

5. The humanized antibody or an antigen binding fragment thereof according to claim 1, wherein the heavy chain variable region has any one amino acid sequence selected from the group consisting of SEQ ID NO: 20, SEQ ID NO: 21, SEQ ID NO: 22 and SEQ ID NO: 23, and
the light chain variable region has any one amino acid sequence selected from the group consisting of SEQ ID NO: 24, SEQ ID NO: 25 and SEQ ID NO: 26.

6. The humanized antibody or an antigen binding fragment thereof according to any one of claims 1 to 5, wherein the humanized antibody further comprises at least one selected from the group consisting of a heavy chain constant region having an amino acid sequence of SEQ ID NO: 27 and a light chain constant region having an amino acid sequence of SEQ ID NO: 28.

7. The humanized antibody or an antigen binding fragment thereof according to any one of claims 1 to 5, wherein the antigen binding fragment is any one selected from the group consisting of Fab, F(ab'), F(ab')₂ and Fv.

8. The humanized antibody or an antigen binding fragment thereof according to any one of claims 1 to 5, wherein the antibody or an antigen binding fragment thereof has an equilibrium dissociation constant(KD) of 2.4×10⁻⁸ M or less.

9. A polynucleotide comprising a base sequence encoding the humanized antibody or an antigen binding fragment thereof according to any one of claims 1 to 5.

10. An expression vector comprising the polynucleotide of claim 9.

11. A host cell comprising the expression vector of claim 10.

12. A method for producing a humanized antibody or an antigen binding fragment thereof comprising culturing the host cell of claim 10.

13. A composition for detecting TM4SF4 comprising the humanized antibody or an antigen binding fragment thereof according to any one of claims 1 to 5.

14. A kit for detecting TM4SF4 comprising the composition for detecting TM4SF4 according to claim 13.

15. A method for detecting TM4SF4 comprising contacting the humanized antibody or an antigen binding fragment thereof according to any one of claims 1 to 5 with a sample to be detected that is expected to contain TM4SF4.

16. A pharmaceutical composition for prevention or treatment of cancer which comprises the humanized antibody or an antigen binding fragment thereof according to any one of claims 1 to 5.

17. The pharmaceutical composition for prevention or treatment of cancer of claim 16, wherein the prevention or treatment of cancer refers to preventing or treating at least one selected from the group consisting of cancer chemoresistance during cancer treatment, cancer chemoresistance after cancer treatment, cancer recurrence, and cancer metastasis.

18. The pharmaceutical composition for prevention or treatment of cancer of claim 16, wherein the cancer is at least one selected from the group consisting of lung cancer, stomach cancer, ovarian cancer, cervical cancer, breast cancer, pancreatic cancer, colorectal cancer, colon cancer, esophageal cancer, skin cancer, thyroid cancer, kidney cancer, liver cancer, head and neck cancer, bladder cancer, prostate cancer, hematologic malignancy, multiple myeloma, acute myeloid leukemia, and malignant lymphoma, thymic cancer, osteosarcoma, fibrous tumor, and brain cancer.

19. A composition for inhibiting the growth of cancer stem cells comprising the humanized antibody or an antigen binding fragment thereof according to any one of claims 1 to 5.

20. A composition for assisting radiation anticancer treatment comprising the humanized antibody or an antigen binding fragment thereof according to any one of claims 1 to 5.

21. The composition for assisting radiation anticancer treatment of claim 20, wherein the humanized antibody or an antigen binding fragment thereof enhances the sensitivity of cancer cells, including cancer stem cells, to radiation.
